# EUROPÄISCHE PATENTSCHRIFT

(11) **EP 3 688 183 B1**
(45) Veröffentlichungstag und Bekanntmachung des Hinweises auf die Patenterteilung: **27.05.2026**
(21) Anmeldenummer: 18774077.4
(22) Anmeldetag: 01.10.2018
(51) Int. Cl.: C12Q 1/22

(54) **VORRICHTUNG UND VERFAHREN ZUR MIKROBIOLOGISCHEN PRÜFUNG VON WASCHMASCHINEN**
DEVICE AND METHOD FOR MICROBIOLOGICAL TESTING OF WASHING MACHINES
DISPOSITIF ET PROCÉDÉ DE CONTRÔLE MICROBIOLOGIQUE DE MACHINES À LAVER

(30) Priorität: 29.09.2017 EP 17193973; 29.09.2017 DE 202017105965 U
(43) Veröffentlichungstag der Anmeldung: 05.08.2020
(73) Patentinhaber: Meducomp GmbH, 15378 Herzfelde (DE)
(72) Erfinder: CYGANEK, Jürgen, 15378 Herzfelde (DE)
(74) Vertreter: Hertin und Partner Rechts- und Patentanwälte PartG mbB
(86) Internationale Anmeldenummer: PCT/EP2018/076642
(87) Internationale Veröffentlichungsnummer: WO 2019/063840

(56) Entgegenhaltungen:
- WO-A1-2011/142824
- WO-A1-2012/092684
- WO-A1-96/06184
- WO-A2-2005/098019
- DE-A1- 3 705 596
- GB-A- 1 055 387
- US-A- 3 346 464
- US-A- 4 743 537
- US-A- 5 856 118
- US-A1- 2008 206 801
- PUBLIC HEALTH AGENCY OF CANADA: "Pathogen Safety Data Sheets: Infectious Substances - Staphylococcus aureus - Canada.ca", 1 January 2011 (2011-01-01), XP055517339, Retrieved from the Internet <URL:https://www.canada.ca/en/public-health/services/laboratory-biosafety-biosecurity/pathogen-safety-data-sheets-risk-assessment/staphylococcus-aureus.html> [retrieved on 20181019]

## Beschreibung

Die Erfindung betrifft einen Bioindikator und ein Verfahren zur Verwendung des Bioindikators zur mikrobiologischen Prüfung von Waschmaschinen. Die erfindungsgemäße Vorrichtung erlaubt eine sichere, zuverlässige, kosteneffiziente und leicht handhabbare mikrobiologische Prüfung der Desinfektionsleistung von Waschmaschinen. Die Erfindung betrifft außerdem eine Verwendung des Bioindikators und einen Kit, der bevorzugt 10 Bioindikatoren umfasst. Insbesondere betrifft die Erfindung einen geschlossenen Bioindikator zur Überprüfung von desinfizierenden Vorgängen wie beispielsweise Spül- oder Waschverfahren, insbesondere in Wasch- oder Spülmaschinen bzw. allen Vorrichtungen, mithilfe derer ein Desinfektionsvorgang durchgeführt bzw. nachgewiesen werden kann. Hierbei kann es sich z.B. um Maschinen mit Desinfektionsprogrammen handeln.

### Hintergrund und Stand der Technik

Zur Vorbeugung vor Krankheiten ist es entscheidend, in sensiblen Umfeldern, wie beispielsweise Krankenhäusern, höchsten Anforderungen an die Hygiene gerecht zu werden. Desinfektionsgeräte wie Wasch- und Spülmaschinen müssen regelmäßig auf ihre Desinfektionsleistung überprüft werden. Auch Spülmaschinen müssen in sensiblen Bereichen ihre Desinfektionsleistung nachweisen.

Funktioniert eine Waschmaschine nicht einwandfrei, kann sie zu einem Ansiedlungspunkt und einer Vermehrungsstätte für Krankheitserreger wie beispielsweise einer Vielzahl von Bakterien werden. Diese können dann beispielsweise von einem eingebrachten, infizierten Gut, wie beispielsweise Krankenhausbettwäsche, auf alle zu waschenden und zu desinfizierenden Güter übergehen. Ein Waschvorgang mit einer defekten oder fehlerhaft bedienten Waschmaschine bietet den Mikroorganismen somit eine ideale Verbreitungsmöglichkeit. Um dies zu verhindern, muss sichergestellt werden, dass die Waschmaschine einwandfrei funktioniert, d.h., dass ihre Desinfektionsleistung den Anforderungen entspricht. Die Desinfektionsleistung wird durch viele Faktoren beeinflusst. Eine unzureichende Desinfektionsleistung kann beispielsweise durch Bedienungsfehler, Fehlprogrammierung oder Fehldosierung von Wirkstoffen oder aber durch ein defektes Desinfektionsgerät hervorgerufen werden.

Deswegen kontrollieren Gesundheitsämter regelmäßig, ob Prüfungen von Desinfektionsgeräten durchgeführt wurden. Auch eine Reihe von Richtlinien von für die Gesundheit zuständigen Instituten, wie zum Beispiel dem Robert-Koch-Institut für Infektionskrankheiten, fordern die regelmäßige Überprüfung solcher Geräte.

Eine Überprüfung der Desinfektionsleistung kann auf mehrere Arten erfolgen. Beispielsweise kann über ein Datenaufzeichnungsgerät direkt die Temperatur des Desinfektionsgeräts aufgezeichnet werden. Bei einigen Desinfektionsverfahren können so Rückschlüsse auf den Desinfektionserfolg gezogen werden. Dieses Verfahren ist jedoch hauptsächlich für rein thermische Desinfektionsverfahren geeignet. Außerdem erfordert diese Methode gegebenenfalls eine teure Nachrüstung der Datenaufzeichnungsgeräte.

Es ist auch möglich, die Desinfektionsleistung mittels sogenannter Abklatschproben zu überprüfen. Dabei wird das zu desinfizierenden Gut nach dem Desinfektionsvorgang mit einem sich in einem Plastikschälchen befindenden Nährboden in Berührung gebracht. Im Labor wird dann untersucht, ob ein Keimwachstum auf dem Nährboden stattgefunden hat. Diese Methode ist jedoch aufwendig, nur von geschultem Fachpersonal durchzuführen und fehleranfällig.

Eine weitere Art der Überprüfung geschieht mittels sogenannter Bioindikatoren. In zahlreichen Ländern wie bspw. Deutschland oder Österreich gibt es hierfür Vorschriften bzw. Richtlinien. Dabei wird ein Keim in einer bestimmten Keimdichte in die Maschine eingebracht. Nach dem Desinfektionslauf, beispielsweise dem Waschgang, wird überprüft, ob und in welcher Größenordnung eine Keimreduktion stattgefunden hat.

Insbesondere Desinfektionsgeräte, welche mit sogenannter Risikowäsche in Berührung kommen, in Krankenhäusern und Altenheim, müssen mindestens halbjährlich überprüft werden.

Bekannt sind Prüfungsmethoden mittels Bioindikatoren durch sogenannte offene Systeme, bei denen man die Keime des Bioindikators direkt in die Waschmaschine einbringt. Beispielsweise kann ein kleines, mit einem geeigneten Indikator versehenes Baumwollsäckchen verwendet werden. Diese Bioindikatoren haben den Vorteil, dass zur Durchführung der Prüfung kein Fachpersonal nötig ist. Solange die Waschmaschine einwandfrei funktioniert, findet eine Keimreduktion statt. Die bekannten Verfahren weisen jedoch mehrere Nachteile auf. Funktioniert ein Gerät nicht einwandfrei, wird das gesamte Waschgut kontaminiert. Somit stellt der Prüfungsvorgang selber ein Gesundheitsrisiko dar. Des Weiteren kann dabei trotz der nicht stattgefundenen Desinfektion eine Verdünnung der Keimdichte auf dem Bioindikator stattfinden, d.h. das Prüfungsergebnis könnte trotzdem positiv sein. Ebenso kann bei diesem Verfahren trotz erfolgreicher Desinfektion der Bioindikator nach der Prüfung durch unsachgemäße Handhabung wieder verkeimt werden und das Prüfungsergebnis fällt negativ aus. Dies hat zu guter Letzt wiederholte Prüfungen und einen mit erhöhten Kosten verbundenen Mehraufwand zur Folge. Die Alltagsnutzung der Waschmaschine zur Desinfektion und Reinigung von Textilien muss dabei länger unterbrochen werden.

Außerdem gibt es in einzelnen Ländern Vorschriften, die mindestens drei Leerläufe der Desinfektionsvorrichtungen wie Geschirrspülern oder Waschmaschinen vorschreiben, nachdem sie zur Desinfektion eingesetzt wurden und das Ergebnis der Kontaminierungsprüfung nicht den Vorschriften genügt. Hierdurch kommt es zu einem großen Verlust an Ressourcen wie bspw. Trinkwasser oder Reinigungsmitteln.

Es sind aus dem Stand der Technik auch wenige geschlossene Bioindikatoren bekannt, welche jedoch einige Nachteile aufweisen. Es wurde beispielsweise in WO 2012/092684 A1 beschrieben, dass ein Bioindikator zur Überprüfung der Desinfektion von sogenannten Containments, abgeschlossenen Räumlichkeiten innerhalb von Laboren oder Reinräumen genutzt werden kann. Der Bioindikator kann entweder säckchenförmig oder als flache Tüte ausgebildet sein und besteht aus einer semipermeablen Hülle um einen Träger mit darauf angebrachten Mikroorganismen. Dieser soll an eine Wandung eines zu desinfizierenden Containments mit einem Klebstreifen angebracht werden. Ein Klebstreifen ist jedoch ungeeignet, um während eines Waschvorgangs an einer Stelle im Innern einer Waschtrommel fixiert zu werden. Dabei könnte vor allem die Wäsche durch den Kleber verunreinigt werden.

Des Weiteren wurden in GB 1055387 Indikatoren zur Überprüfung eines Sterilisationsprozesses in Form einer semipermeablen Tüte vorgeschlagen, welche im Inneren auf einem Träger eine definierte Anzahl eines Testorganismus aufweist. Nach der Sterilisation wird die Tüte in Wasser gesetzt, wodurch in Verbindung mit einem enthaltenen Wachstumsmedium das Wachstum rückständiger Mikroorganismen in Gang gesetzt werden kann. Daher ist diese Tüte für den Test von Waschvorgängen in Verbindung mit Wasser nicht geeignet, da hier ebenfalls das Wachstum angeregt werden könnte. Eine Verwendung in einer Wasch- oder Spülmaschine oder ein dahingehendes Verfahren werden konsequenterweise auch nicht offenbart. Ein Bioindikator, welches ebenfalls nach der Sterilisation in ein Wasserbad gegeben wird, um ein Wachstum etwaiger verbliebener Mikroorganismen anzuregen wird in US 3346474 offenbart.

Auch ein Indikator zur Überprüfung von Desinfektionsprozessen aus hydrophilem Material ist aus WO 96/06184 bekannt, ein Verfahren zur Verwendung für Waschmaschinen wurde jedoch nicht beschrieben.

Auch bekannt ist ein biologischer Indikator zur Überprüfung von Desinfektionsvorgängen unter Verwendung gasförmiger Desinfektionsmittel, z. B. Wasserstoffperoxid (US Auch ein Bioindikator zur Überprüfung der Desinfektionsleistung bei Waschvorgängen ist bekannt, welcher jedoch in einem unpraktischen und wenig kompakten, unflexiblen und dosenförmigen Behälter untergebracht ist. Daher ist der Materialaufwand sehr hoch und es wird bei einem Waschvorgang viel Platz durch den Indikator selber eingenommen. Auch eine Wahrscheinlichkeit für eine Schädigung von mitgewaschenem Waschgut und/oder der Waschmaschine selber ist bei Verwendung dieses Indikators ist deutlich erhöht.

Allen vorgenannten Indikatoren ist ebenfalls gemein, dass keine Sets von Indikatoren zur Überprüfung von Waschmaschinen verwendet werden. Daraus ergeben sich Nachteile bei der Auswertung, wie eine höhere Fehleranfälligkeit und eine geringere Sicherheit einer Überprüfung, weil z. B. nicht effizient mehrere Referenzkeime gleichzeitig bei einer Überprüfung verwendet werden können.

Auch ein Verfahren für eine Überprüfung, welche besonders benutzerfreundlich ist und das Einsenden der Indikatoren an eine Prüfstelle umfassen, wird nicht beschrieben. Die genannten Indikatoren sind für einen effizienten Versand nicht geeignet.

### Aufgabe der Erfindung

Aufgabe der Erfindung ist eine Vorrichtung und ein Verfahren bereitzustellen, welches die Nachteile des Standes der Technik nicht aufweist.

Der Erfindung liegt insbesondere die Aufgabe zugrunde, ein Verfahren sowie eine Vorrichtung zur effizienten Überprüfung der Desinfektionsleistung von Desinfektionsgeräten, insbesondere Waschmaschinen, bereitzustellen, wobei das Verfahren und die Vorrichtung einfach und ohne die Anwesenheit von Fachpersonal anwendbar ist, und dabei die Gefahr einer fehlerhaften Durchführung minimiert bzw. ausschließt und besonders sicher ist. Vor allem soll die mögliche Kontamination des gesamten Waschguts und daraus möglicherweise resultierende weitere Kontaminationen bei einer Überprüfung sowie das Risiko eines verfälschten Prüfungsergebnisses minimiert oder verhindert werden.

### Kennzeichnung der Erfindung

Erfindungsgemäß wird die Aufgabe durch eine Vorrichtung gemäß den unabhängigen Patentansprüchen gelöst. Die abhängigen Patentansprüche stellen bevorzugte Ausführungsformen der Erfindung dar.

In einem Aspekt betrifft die Erfindung einen Bioindikator zur mikrobiologischen Prüfung von Waschmaschinen, wobei
- der Bioindikator einen Träger umfasst,
- der Träger Referenzkeime aufweist, wobei 2008/0206801 A1). Dieser weist z. B. einen Träger aus Metall auf, wodurch eine Verwendung bei flüssigkeitsbasierten Desinfektionsvorgängen nicht infrage kommt. Auch eine Vorrichtung zur Überprüfung eines Desinfektionsvorgangs mit einer zusätzlichen Schutzschicht um eine Membran wird beschrieben. Dieser Indikator ist daher nicht einfach und effizient aufgebaut und kostenintensiv in der Herstellung.

Auch ein Bioindikator zur Überprüfung der Desinfektionsleistung bei Waschvorgängen ist bekannt (WO 2005/098019), bei welchem ein Mikroorganismus auf einen Träger aufgebracht wird und durch eine semipermeable Membran oder Filtermaterialien abgetrennt werden. Der Bioindikator wird jedoch in einem unpraktischen und wenig kompakten, unflexiblen und dosenförmigen Behälter untergebracht. Daher ist der Materialaufwand sehr hoch und es wird bei einem Waschvorgang viel Platz durch den Indikator selber eingenommen. Auch eine Wahrscheinlichkeit für eine Schädigung von mitgewaschenem Waschgut und/oder der Waschmaschine selber ist bei Verwendung dieses Indikators ist deutlich erhöht. DE3705596 A1 beschreibt ebenfalls einen Bioindikator zur Überprüfung eines Desinfektionsvorgangs bei einem Waschvorgang. Neben einem Bioindikator in Form eines Trägers mit Mikroorganismen, welcher von einer semipermeablen Membran umhüllt ist, umfasst der Bioindikator einen Temperatursensor zur Überwachung des Waschvorgangs. Ber Bioindikator und der Sensor sind dabei durch ein weiteres Material ummantelt, welches dem Bioindikator Stabilität verleiht.

WO2011/142824 A1 offenbart einen Bioindikator für die Überprüfung eines Sterilisationsprozesses in einer Sterilisationskammer, welcher einen Träger umfasst, auf welchem Referenzkeime aufgebracht sind und welcher von einer Hülle umgeben ist, welche sich aus zwei Membranen zusammensetzt.

Allen vorgenannten Indikatoren ist ebenfalls gemein, dass keine Sets von Indikatoren zur Überprüfung von Waschmaschinen verwendet werden. Daraus ergeben sich Nachteile bei der Auswertung, wie eine höhere Fehleranfälligkeit und eine geringere Sicherheit einer Überprüfung, weil z. B. nicht effizient mehrere Referenzkeime gleichzeitig bei einer Überprüfung verwendet werden können.

Auch ein Verfahren für eine Überprüfung, welche besonders benutzerfreundlich ist und das Einsenden der Indikatoren an eine Prüfstelle umfassen, wird nicht beschrieben. Die genannten Indikatoren sind für einen effizienten Versand nicht geeignet.

### Aufgabe der Erfindung

Aufgabe der Erfindung ist eine Vorrichtung und ein Verfahren bereitzustellen, welches die Nachteile des Standes der Technik nicht aufweist.

Der Erfindung liegt insbesondere die Aufgabe zugrunde, ein Verfahren sowie eine Vorrichtung zur effizienten Überprüfung der Desinfektionsleistung von Desinfektionsgeräten, insbesondere Waschmaschinen, bereitzustellen, wobei das Verfahren und die Vorrichtung einfach und ohne die Anwesenheit von Fachpersonal anwendbar ist, und dabei die Gefahr einer fehlerhaften Durchführung minimiert bzw. ausschließt und besonders sicher ist. Vor allem soll die mögliche Kontamination des gesamten Waschguts und daraus möglicherweise resultierende weitere Kontaminationen bei einer Überprüfung sowie das Risiko eines verfälschten Prüfungsergebnisses minimiert oder verhindert werden.

### Kennzeichnung der Erfindung

Erfindungsgemäß wird die Aufgabe durch eine Vorrichtung gemäß den unabhängigen Patentansprüchen gelöst. Die abhängigen Patentansprüche stellen bevorzugte Ausführungsformen der Erfindung dar.

In einem Aspekt betrifft die Erfindung einen Bioindikator zur mikrobiologischen Prüfung von Waschmaschinen, wobei
- der Bioindikator einen Träger umfasst,
- der Träger Referenzkeime aufweist, wobei
- der Träger durch eine semipermeable Membran umschlossen ist, wie in Anspruch 1 beschrieben.

Unter Bioindikator versteht man im Sinne der Erfindung bevorzugt eine Vorrichtung, welche über den Vergleich der Anzahl von Mikroorganismen in einem räumlich begrenzten Bereich vor und nach einem zu prüfenden Desinfektionsvorgang einen Aufschluss über den Erfolg des Desinfektionsvorgangs zulässt. Räumlich begrenzter Bereich bezeichnet im Sinne der Erfindung bevorzugt eine Fläche mit einer Ausdehnung zwischen 0,25 cm² und 100 cm² und umfasst bevorzugt einen Wesentlichen Teil des Bioindikators. Mit Desinfektionsvorgang ist bevorzugt ein Vorgang gemeint, vormals pathogene Mikroorganismen in einen Zustand zu versetzen, in dem sie nicht mehr pathogen sind. Besonders bevorzugt dient der Desinfektionsvorgang dem Abtöten der Mkroorgansimen. Mit pathogen ist bevorzugt potentiell krankheitserregend gemeint. Besonders bevorzugt ist mit Desinfektionsvorgang im Sinne der Erfindung ein Waschvorgang einer Waschmaschine oder ein Spülvorgang einer Spülmaschine gemeint. Der Desinfektionsvorgang kann thermisch und/oder chemisch vorgenommen werden. Durch die Messung der Dichte von Mikroorganismen als Referenzkeimen, zum Beispiel durch die Messung der Dichte von Bakterien vor und nach einer Desinfektionshandlung, kann der Erfolg der Desinfektionshandlung gemessen werden. Mit Dichte bzw. Keimdichte ist bevorzugt die Anzahl pro Fläche gemeint, besonders bevorzugt ist die Anzahl pro cm² gemeint. Es kann aber auch bevorzugt sein, dass die Anzahl der Keime pro Bioindikator gemeint ist. Erfolg bezeichnet bevorzugt eine Keimreduktion um mehrere Größenordnungen, also bevorzugt um einen Faktor von mehreren Zehnerpotenzen. Typischerweise soll bei einer erfolgreichen Keimreduktion eine Reduktion der Keimdichte um bevorzugt mindestens drei Größenordnungen oder Log-Stufen, insbesondere um mindestens vier Größenordnungen oder Log-Stufen, ganz besonders bevorzugt um mindestens fünf Größenordnungen oder Log-Stufen, in manchen Fällen bevorzugt mindestens sechs Größenordnungen oder Log-Stufen erreicht werden. Zum Nachweis der Keimreduktion bzw. zur Messung der Keimdichte können gängige Verfahren, wie beispielsweise bevorzugt die Gram-Färbung, lichtmikroskopische Untersuchungen, aber auch das Anwachsen einer Kultur auf einem Nährboden, verwendet werden.

Unter mikrobiologischer Prüfung wird bevorzugt eine Prüfung bezüglich Mikroorganismen, also Organismen, die sehr klein sind und aus einer bis wenigen Zellen bestehen, bezeichnet. Selbstverständlich können auch vielzellige Pathogene wie Würmer gemeint sein. Unter Mikroorganismen werden im Sinne der Erfindung bevorzugt Bakterien, Pilze und Viren bezeichnet. Hierbei sind insbesondere pathogene Mikroorganismen von Interesse. Bakterien sind im Sinne der Erfindung alle Prokaryoten, bei denen die DNA frei im Cytoplasma vorliegt. Bakterien gehören im Sinne der Erfindung bevorzugt zu den folgenden Stämmen: Actinobacteria, Firmicutes, Tenericutes, Aquificae, Bacteroidetes, Fibrobacteres, Chlorobi, Chlamydiae, Deinococcus-Thermus, Fusobacteria, Gemmatimonadetes, Nitrospirae, Planctomyceten, Verrucomicrobia, Chlamydiae, Proteobacteria, Spirochaetes, Synergistetes, Acidobacteria, Chloroflexi, Chrysiogenetes, Cyanobakterium, Deferribacteres, Dictyoglomi, Thermodesulfobacteria und/oder Thermotogae.

Unter den pilzartigen Mikroorganismen sind bevorzugt folgende Pilze zu verstehen: Hefen, wie beispielsweise Malassezia furfur und Candida albicans, Dermatophyten, verschiedene Aspergillus-Arten, zum Beispiel Aspergillus fumigatus. Außerdem können insbesondere Pilze der Gattung Cryptococcus, Rhizopus, Coccidioides und/oder Histoplasma pilzartige Mikroorganismen sein.

Viren sind bevorzugt infektiöse Partikel, die sich als Virionen außerhalb von Zellen (extrazellulär) durch Übertragung verbreiten, aber als Viren vor allem innerhalb einer geeigneten Wirtszelle (intrazellulär) vermehren können. Die bei der mikrobiologischen Prüfung im Sinne der Erfindung relevanten Viren sind bevorzugt Viren der Gattungen Orthopoxvirus, Parapoxvirus, Molluscipoxvirus, Simplexvirus, Varicellovirus, Cytomegalovirus, Reseolovirus, Lymphocryptovirus, Rhadinovirus, Orthohepadnavirus, Rubiviren, Flavivirus, Alphacoronavirus, Torovirus, Deltaretrovirus, Lentivirus, Bornavirus, Orthobunyavirus, Phlebovirus, Nairovirus, Hantavirus, Influenzavirus A, Influenzavirus B, Influenzavirus C, Avulavirus, Morbillivirus, Henipavirus, Rubulaviren, Pneumovirus, Metapneumovirus, Vesiculovirus, Mastadenovirus, Polyomavirus, Dependovirus, Erythrovirus, Rotavirus, Coltivirus, Norovirus, Sapovirus, Hepevirus, Enterovirus, Hepatovirus und/oder Rhinovirus.

Unter Prüfung ist bevorzugt eine Kontrolle auf Vorhandensein eines Mikroorganismus und/oder von Teilen eines Mikroorganismus zu verstehen. Es ist bevorzugt, dass bei der Kontrolle auf Vorhandensein die Anzahl und/oder die Dichte der Mikroorganismen bestimmt werden kann. Bevorzugt kann die Anzahl und/oder die Dichte vor und nach dem zu überprüfenden Desinfektionsvorgang und somit die Keimreduktion bestimmt werden. Es kann auch bevorzugt sein, dass vor dem Desinfektionsvorgang eine bekannte Anzahl an Mikroorganismen vorhanden ist, und nach dem Desinfektionsvorgang die Anzahl der Mikroorganismen bestimmt werden kann. Die Überprüfung kann beispielsweise durch Gram-Färbung, lichtmikroskopische Untersuchungen, aber auch das Anwachsen einer Kultur auf einem Nährboden stattfinden.

Eine Waschmaschine dient der Reinigung von Waschgütern. Bei diesem Reinigungsprozess wirken bevorzugt mechanische Kräfte und Wasser kombiniert auf das zu waschende Gut ein. Dem Waschwasser werden bevorzugt Waschmittel zugegeben, die bevorzugt ebenso wie die Temperatur des Waschwassers an das Material des zu waschenden Guts und dessen Verschmutzungsgrad und Verschmutzungsart angepasst werden. Waschmaschinen sind im klinischen Bereich verbreitet, um Bekleidung und andere textile Erzeugnisse zu reinigen und bevorzugt zu desinfizieren.

Unter Träger sind bevorzugt flächige Elemente zu verstehen, welche zur Aufnahme von Referenzkeimen geeignet sind. Ein Träger kann verschiedenste Beschaffenheit aufweisen, beispielsweise kann er starr aber auch flexibel sein. Ebenso sind verschiedenste Geometrien, beispielsweise rund, viereckig, insbesondere quadratisch, aber ebenso andere geometrische Formen denkbar. Unter flächig ist bevorzugt zu verstehen, dass das Element in zwei Dimensionen eine große Ausdehnung und in der dritten eine geringe Ausdehnung hat. Beispielsweise kann das Element in zwei Dimensionen eine Ausdehnung von jeweils mindestens 1 cm aufweisen, und dabei eine Dicke von nur wenigen Millimetern oder sogar kleiner als 1 mm aufweisen.

Verschiedenste Materialien sind als Trägermaterial denkbar. Es sind beispielsweise textile Materialien aus Naturfasern wie Baumwolle bevorzugt, aber auch Polymere sind als bevorzugtes Trägermaterial denkbar.

Bevorzugt ist, dass der Träger vor dem Aufbringen der Referenzkeime im Wesentlichen steril ist, d.h. dass er von Mikroorganismen frei ist. Frei bedeutet im Sinne der Erfindung, dass auf dem Träger ein Mikroorganismus mit einer Wahrscheinlichkeit von 10⁻¹, bevorzugt 10⁻², besonders bevorzugt 10⁻³, ganz besonders bevorzugt 10⁻⁴ oder kleiner vorhanden ist. Es kann auch bevorzugt sein, dass auf dem Träger ein Mikroorganismus mit einer Wahrscheinlichkeit von 10⁻⁵, 10⁻⁶ oder kleiner vorhanden ist.

Es kann auch bevorzugt sein, dass der Träger die nach innen zeigende Seite der Membran ist. Nach innen zeigend meint bevorzugt die Seite der Membran, auf welcher sich die Referenzkeime vor der Prüfung befinden. So ist die Membran bevorzugt eine nach außen geschlossene Hülle, deren Innenseite als Träger fungiert und die Referenzkeime aufweist.

Unter Referenzkeime sind bevorzugt Mikroorganismen zu verstehen, die kontrolliert und ohne Gesundheitsrisiko für das beteiligte Fachpersonal auf den Träger aufgebracht werden können. Kontrolliert bedeutet, dass beim Aufbringen eine gewünschte Keimdichte erreicht werden kann. Es kann bevorzugt sein, eine Keimdichte von beispielsweise bevorzugt mindestens 10⁷ aufzubringen. Es kann auch bevorzugt sein eine Keimdichte von bis zu 10⁷ aufzubringen. Gleichzeitig sollte ihre Anzahl bzw. Keimdichte bevorzugt unter dem zu prüfenden Desinfektionsverfahren in vorhersagbarer und fehlerfrei überprüfbarer Weise reduziert werden. Darunter ist bevorzugt zu verstehen, dass eine Keimreduktion um einen Faktor von mindestens 10³, bevorzugt 10⁴, besonders bevorzugt 10⁵ und ganz besonders bevorzugt 10⁶ stattfindet. Es können thermische oder nicht-thermische Desinfektionsverfahren mittels Referenzkeimen überprüft werden.

Bevorzugt ist, dass der Referenzkeim im Falle einer möglichen Exposition nur geringes pathogenes Risiko aufweist. Exposition bedeutet vor allem, dass eine Person mit dem Referenzkeim insbesondere in direkten Kontakt kommt und ihn beispielsweise dabei aufnimmt. Eine Aufnahme kann insbesondere oral, inhalativ, dermal, intravenös, intramuskulär oder intraperitoneal erfolgen. Es ist bevorzugt, dass die Referenzkeime auf ähnliche Weise auf den Desinfektionsvorgang reagieren wie die typischerweise natürlich auftretenden und zu desinfizierenden Mikroorganismen bzw. mit ihnen identisch sind. Bevorzugt sind mit Referenzkeimen Bakterien, Pilze und/oder Viren gemeint. Bevorzugte Beispiele für Bakterien, Viren und/oder Pilze sind: Aspergillus fumigatus, Bacillus subtilis, Bacteroides fragilis, Candida albicans, Candida krusei, Candida tropicalis, Corynebacterium renale, Escherichia coli, Enterobacter casseluiflavus, Enterobacter hormeachei, Enterococcus faecium, Haemophilus influenzae, Klebs pneumoniae, Micrococcus luteus, Enterococcus faecalis, Neisseria gonorrhoeae, Proteus mirabilis, Proteus vulgaris, Pseudomonas aeruginosa, Staphylococcus epidermidis, Staphylococcus aureus, Streptococcus pneumoniae, Streptococcus agalactiae, Mycoplasma hominis, Pseudomonas aeruginosa, Salmonella enterica, Staphylococcus saprophyticus, Streptococcus pyogenes und/oder Ureaplasma parvum.

Unter Membran ist insbesondere eine dünne Schicht eines Materials gemeint, welche den Stofftransport durch diese Schicht beeinflusst. Die Semipermeabilität kann vor allem durch die Struktur der Membran, insbesondere durch die Geometrie von Aussparungen bzw. Poren in der Struktur erreicht werden. Diese können beispielsweise so klein sein, dass sie nicht von Referenzkeimen passiert werden können.

Die Membran hat bevorzugt eine Dicke zwischen 0,005 und 1 mm, besonders bevorzugt eine Dicke zwischen 0,01 und 0,5 mm und insbesondere zwischen 0,05 und 0,1 mm. Diese Membrandicke ist im Zusammenspiel mit dem Membranmaterial besonders geeignet, gewünschte Materialeigenschaften, wie Flexibilität und Stabilität zu erreichen. Auch ergeben sich in Verbindung mit der bevorzugten Porengröße auf synergistische Weise eine überraschend gute Durchlässigkeit für Wasser/Desinfektionsflüssigkeit einerseits und Undurchlässigkeit für die Referenzkeime im Inneren des Indikators andererseits. Die erfindungsgemäße Aufgabe kann mit dieser bevorzugten Ausführungsform besonders fehlerfrei, effizient, sehr gut reproduzierbar und sicher gelöst werden.

Unter semipermeable Membran ist bevorzugt eine Membran gemeint, welche durchlässig für Wasser und in Wasser gelöste Desinfektionswirkstoffe ist, jedoch undurchlässig für die Referenzkeime.

Bevorzugt können Membran Polymermembranen oder keramische Membranen sein. Beispiele für bevorzugte Gruppen von Materialien zur Herstellung von Membranen sind Zeolithe und/oder Polyamide.

Die semipermeable Membran ist bevorzugt mechanisch, chemisch und thermisch stabil, gleichzeitig flexibel.

Es ist bevorzugt, dass die semipermeable Membran des Bioindikators Poren aufweist, bevorzugt muss die Größe der Poren kleiner sein als der Durchmesser der abzutrennenden Bestandteile, welche bevorzugt die Referenzkeime sind. Mit Größe der Poren ist bevorzugt der Durchmesser der Poren gemeint. Die Poren können beispielsweise oder bevorzugt eine Größe von 0,5 µm aufweisen.

Es werden bevorzugt je nach Anwendung unterschiedliche Membrangeometrien verwendet. Es kann bevorzugt sein, eine Flachmembran zu verwenden, d.h. bevorzugt, dass poröse Folien aus Polymer oder keramische Scheiben, welche gerakelt oder gegossen werden, als Membrangeometrie verwendet werden. Beim Rakeln werden Polymerlösungen bevorzugt mittels einer Metall-Rakel ausgestrichen und durch eine Phaseninversion zu einer Flachmembran gefällt. Als Phaseninversion wird eine Umkehr der Phase bezeichnet. Die Phase bezeichnet in erster Linie die in einem räumlichen Bereich, welcher eine homogene chemische Zusammensetzung aufweist, homogen vorliegenden bestimmenden physikalischen Parameter, wie bspw. den Aggregatzustand. Mit Fällung oder Präzipitation wird bevorzugt in der Chemie das Ausscheiden des in einer Lösung gelösten Stoffes bezeichnet. Solche Membranen sind besonders kostengünstig, dabei effektiv. Es kann auch bevorzugt sein, kapillarartige Hohlfasermembranen zu verwenden. Unter einer Hohlfaser versteht man in der Hauptsache eine Faser, welche zylinderförmig ist und einen oder mehrere durchgängige Hohlräume in ihrem Querschnitt aufweist. Im Allgemeinen versteht man unter kapillarartig Röhrchen oder Hohlräume mit sehr kleinen Innendurchmessern, wodurch physikalische Effekte, insbesondere der Kapillareffekt, auftreten. Physikalische Begriffe wie "Kapillareffekt" sind insbesondere so zu verstehen, wie in der Fachliteratur, beispielsweise in "H. Schubert: Kapillarität in porösen Feststoffsystemen, Springer Verlag, Berlin" beschrieben wird. Solche Membranen sind bezüglich ihrer Filterfunktion besonders leistungsstark. Es kann auch bevorzugt sein, sogenannte Wickelmodule als Membran zu verwenden, d.h. bevorzugt, dass zwei Flachmembranlagen, die durch ein Gewebe voneinander getrennt spiralförmig aufgewickelt werden, verwendet werden. Auf diese Weise können besonders zuverlässig abzutrennende Bestandteile unterschiedlichster Größen gefiltert werden. Es kann auch bevorzugt sein, Multikanalelemente zu verwenden, d.h. bevorzugt, extrudierte, keramische Zylinder oder Platten, welche durch innen beschichtete Kanäle durchströmt werden. Extrusion beschreibt vor allem Vorgänge, bei denen feste bis dickflüssige, härtbare Materialien unter Druck aus einer formgebenden Öffnung herausgedrückt werden. Solche Membranen sind besonders zuverlässig. Bevorzugt können auch Composite-Membran zum Einsatz kommen. Bei diesen Membranen wird bevorzugt auf eine poröse Trägerschicht eine aktive Membranschicht aufgetragen. Unter aktive Membranschicht kann bevorzugt eine Membranschicht gemeint sein, welche außer durch ihre Geometrie auch durch zusätzliche Effekte, beispielsweise durch chemische Prozesse, eine Filterfunktion erfüllt. Es kann aber auch vorteilhafterweise gemeint sein, dass Energie hinzugeführt werden muss, um die Filterfunktion der Membran zu realisieren. Solche Membranen können eine Verbesserung der Filtercharakteristik bewirken und weisen oftmals synergistische Effekte auf, da ihre Filtereigenschaften besser sind, als allein durch die Summe der durch die Geometrie verursachten Filterfunktion und der durch die zusätzlichen Effekte verursachten Filterfunktion zu erwarten ist.

Ein Träger, der durch eine semipermeable Membran (zumindest teilweise) umschlossen ist, bezeichnet bevorzugt einen Träger, der von Membran wie von einer Hülle allseitig umgeben ist, so dass die von ihm getragenen Referenzkeime nicht auf die Außenseite der Membran gelangen können. Es kann auch bevorzugt sein, dass der Träger die Innenseite der Membran selber ist, so dass der von der Membran umschlossene Träger eine von der Membran bereitgestellte Fläche auf der Innenseite der Membran ist, welche von den Teilen der Membran, welche nicht zu dieser Fläche gehören, wie von einer Hülle allseitig umgeben ist. Erfindungsgemäß kann es sich hierbei auch um ein Baumwollsäckchen handeln. Es ist dabei bevorzugt, dass sich die Membran der Form des Trägermaterials dabei weitgehend anpasst. So kann ein besonders kompakter und platzsparender Bioindikator hergestellt werden. Die Referenzkeime sind bevorzugt wie der Tee in einem Teebeutel der Wasch/Desinfektionsflüssigkeit zugänglich und können mit dieser wechselwirken (Desinfektion), können aber gleichzeitig nicht aus dem Inneren der Membran nach außen gelangen.

Das Verschließen der Membran nach außen hin kann bevorzugt durch ein Schweiß- oder Klebeverfahren realisiert werden. Schweißen meint bevorzugt ein Verfahren, bei dem Wärmezufuhr bis zum Schmelzen des Werkstoffs oder Wärmezufuhr und bevorzugt zusätzlich eine Druckeinwirkung auf das Werkstück erfolgen.

Durch diese bevorzugte Ausführungsform des Bioindikators wird eine besonders einfache, robuste und kostengünstig herzustellende Vorrichtung bereitgestellt. Es kann so eine besonders einfache Herstellung des Bioindikators erfolgen, bei der zusätzlich Material eingespart wird.

Die Erfindung löst auf überraschende Weise die sich aus den Nachteilen des Standes der Technik ergebende Aufgabe. Die mikrobiologische Prüfung von Waschmaschinen mittels Bioindikator kann ohne Fachpersonal umgesetzt werden. Dabei kann es, anders als im Stand der Technik, nicht dazu kommen, dass aufgrund eines fehlerhaften Desinfektionsvorgangs das gesamte Waschwasser mit pathogenen Mikroorganismen verseucht wird. Ebenso wenig kann es zu fehlerhaften Prüfergebnissen kommen, weil die Referenzkeime während des Waschvorgangs abgewaschen aber nicht unschädlich gemacht werden. Auch können die bevorzugten Bioindikatoren nicht durch bei falscher Handhabung nachträglich aufgebrachten Keimen zu fehlerhaften Prüfergebnissen führen. Es war überraschend, dass all die Nachteile bekannter Bioindikatoren durch die bevorzugte Ausführungsform überwunden werden konnten. Durch die erhöhte Sicherheit und Zuverlässigkeit bei der mikrobiologischen Prüfung von Waschmaschinen sowie der einfachen Handhabung des Bioindikators kann eine nachhaltige und kostensparende Prüfung erfolgen, welche zu keinen Unterbrechungen der täglichen Routinewaschvorgänge führt.

In einer bevorzugten Ausführungsform der Erfindung umfasst der Bioindikator zur mikrobiologischen Prüfung von Waschmaschinen
- einen Träger umfasst, wobei der Träger Referenzkeime aufweist, und
- eine semipermeable Membran, wobei
der Träger durch die semipermeable Membran umschlossen ist, wobei die Membran eine Polymermembran ist und Poren einer Größe zwischen 0,1 µm und 2 µm aufweist.

Es war überraschend, dass eine Polymermembran mit diesen Porengrößen zur Verfügung gestellt werden konnte, die darüber hinaus die erfindungsgemäße Aufgabe besonders gut löst. Es war außerdem äußerst überraschend, dass eine solche Polymermembran in Kombination mit diesen Porengrößen eine besonders effektive Filterung der Referenzkeime einerseits und eine Durchlässigkeit für Wasser bzw. Desinfektionsflüssigkeit anderseits aufweist. Während Vorrichtungen des Standes der Technik kaum reproduzierbare Ergebnisse gestatteten, so dass in der Praxis oft mehrere Vorrichtungen parallel genutzt werden mussten, erlaubt die bevorzugte, erfindungsgemäße Vorrichtung den materialschonenden Einsatz weniger oder nur einer Vorrichtung, welche gleichsam reproduzierbare Ergebnisse zur Verfügung stellt. Diese Vorzugsvariante stellt eine Abkehr vom technisch Üblichen dar.

In einer bevorzugten Ausführungsform der Erfindung ist die die Membran, bevorzugt die Polymermebran hydrophob. Somit ist ein sofortiges durchnässen der Membran mit der Desinfektionsflotte bzw. der Desinfektions-/Waschflüssigkeit. Mit hydrophob werde bevorzugt Substanzen charakterisiert, welche sich im Wesentlichen nicht mit Wasser mischen und dieses bspw. auf ihrer Oberflächen zumindest teilweise "abperlen" lassen. Beispielsweise kann die Hydrophobizität nach IUPAC-( International Union of Pure and Applied Chemistry) Definition definiert sein, wonach diese den Zusammenschluss unpolarer Gruppen oder Moleküle in einer wässrigen Umgebung darstellt. Hydrophobie lässt sich ebenso bevorzugt durch eine Hydrophobizitätsskala beschreiben. Z. B. kann eine hydrophobe Membran einen Octanol-Wasser-Verteilungskoeffizient größer 1, insbesondere deutlich größer 1 aufweisen. Es kann ebenso bevorzugt der Kontaktwinkel zur Beschreibung herangezogen werden. So wird insbesondere der Winkel bezeichnet, den ein Flüssigkeitstropfen (Wassertropfen) auf der Oberfläche einer Substanz zur Oberfläche bildet. Bevorzugt bedeutet ein Kontaktwinkel von ca. 90 ° Hydrophobie, ebenso ein Winkel von über 90 °. Ein Fachmann weiß, wie er das Membranmaterial präparieren muss, damit es im Wesentlichen hydrophob, z. B. durch Verwendung und/oder Beschichtung geeigneten Materials (z. B. Polytetrafluorethylen, Wachs und/oder Paraffin) und eine besondere Strukturierung (Beispiel Lotuspflanze) Dies ist insbesondere wichtig, da die Desinfektionsflotte bzw. die Desinfektions-/Waschflüssigkeit daher sofort an den eingebrachten Bioindikator gelangt. In der Vergangenheit ist man stets davon ausgegangen, dass eine Membran hydrophil sein muss für einen guten Durchfluss mit Wasser und/oder der Desinfektionsflüssigkeit. Die besondere Eignung einer hydrophoben Membran zur Überprüfung einer Desinfektionsleistung einer Waschmaschine ist daher besonders überraschend. Es können sich synergistische Effekte ergeben gemeinsam mit dem verwendeten Membranmaterial und/oder der Porengröße.

In einer bevorzugten Ausführungsform ist die Polymermembran eine flexible Polymermembran. Eine flexible Polymermembran ist besonders geeignet für die Verwendung in einer Waschmaschine gemeinsam mit einem zu desinfizierenden Waschgut. Durch die Flexibilität wird das Waschgut nicht beeinflusst und die auch die Waschvorrichtung selber wird mechanisch weniger beansprucht, was die Langlebigkeit der Waschvorrichtung und des Waschguts erhöht. Auch bei einem voll mit Waschgut befülltem Waschraum (Waschtrommel) kann ein solcher Indikator sich besonders gut an die gegebenen Platzverhältnisse anpassen.

Eine flexible Polymermebran ist dabei bevorzugt fluid, veränderlich und/oder gummiartig, dabei im Wesentlichen formstabil auch unter hohen Temperaturen. Das Polymer ist dabei trotz der im Wesentlichen Formstabilität anpassungsfähig und biegbar. Bevorzugt ist das flexible Polymer nachgiebig. Die gewünschten Eigenschaften können, wie der Fachmann weiß, durch bevorzugte Dicken, Materialzusammensetzungen und/oder Strukturierungen erreicht werden. In einer bevorzugten Ausführungsform ist die Polymermembran eine Polymerfolie.

In einer bevorzugten Ausführungsform ist der Bioindikator flächig. Insbesondere ist der Indikator im Wesentlichen in einer Ebene ausgedehnt und nur unwesentlich entlang einer Dicke senkrecht zu dieser Ebene. Ein solcher Indikator nutzt besonders effektiv den Platz in einer Waschmaschine zwischen dem Waschgut. Insbesondere die Anpassungsfähigkeit an die Waschmaschine und das Waschgut wird synergistisch gesteigert.

Begriffe wie im Wesentlichen, ungefähr, ca. etc. beschreiben bevorzugt einen Toleranzbereich von weniger als ± 20%, bevorzugt weniger als ± 10%, besonders bevorzugt weniger als ± 5% und insbesondere weniger als ± 1%. Ähnlich beschreibt bevorzugt Größen die ungefähr gleich sind. Teilweise beschreibt bevorzugt zu mindestens 5 %, besonders bevorzugt zu mindestens 10 %, und insbesondere zu mindestens 20 %, in einigen Fällen zu mindestens 40 %.

Bevorzugt weist der Indikator eine Dicke von weniger als 1 mm, besonders bevorzugt weniger als 0,5 mm und insbesondere weniger als 0,2 mm auf. Ein solcher Träger ist besonders platzsparend. Die Fachwelt ist bislang davon ausgegangen, dass ein solcher Indikator nicht stabil ist und eine Schutzhülle benötigt. Die Erfindung konnte dieses Vorurteil überraschend überwinden.

Bevorzugt weist der flächige Indikator ein rechteckiges Format mit Kantenlängen kleiner als 4 cm, besonders bevorzugt kleiner als 3 cm auf. Ein solch kompakter Indikator ist für die Überprüfung von Waschmaschinen, insbesondere gemeinsam mit Waschgut, besonders geeignet. Es war überraschend, dass ein solcher bevorzugter Indikator eine besonders sichere mikrobiologische Prüfung erlaubt. Sicher bedeutet, dass die Detektion der Keime fehlerfreier als im Stand der Technik erfolgt und weiterhin, dass das Waschgut nicht negativ beeinflusst wird.

In einer bevorzugten Ausführungsform der Erfindung ist der Träger beidseitig durchlässig für eine Desinfektionsflüssigkeit. Insbesondere mit der umgebenden, durchlässigen Membran ergeben sich so überraschende Verbesserungen gegenüber dem Stand der Technik. So kann eine besonders effektive Prüfung vorgenommen werden, da ein beidseitig durchlässiger Träger von der Desinfektionsflüssigkeit in höherem Maße durchflossen wird und dadurch die Wahrscheinlichkeit für falsche Resultate überraschend verringert oder ausgeschlossen wird.

In einer bevorzugten Ausführungsform ist der Bioindikator konfiguriert für mikrobiologische Prüfung von Waschmaschinen, bevorzugt gemeinsam mit einem Waschgut. Das bedeutet, dass er spezielle Eigenschaften für die Überprüfung von Waschmaschinen gemeinsam mit einem Waschgut aufweist. Insbesondere ist flächig, weist ein kompaktes Format auf und ist flexibel, dabei gleichzeitig stabil. Ebenso ist er bevorzugt nichthaftend. Wenn eine Überprüfung gemeinsam mit Waschgut stattfindet, kann die Überprüfung besonders effizient sein, da der übliche Waschvorgang nicht unterbrochen werden muss.

In einer bevorzugten Ausführungsform sind die Referenzkeime des Bioindikators ausgewählt aus dem Stamm der Firmicutes. Es ist besonders bevorzugt, einen Bioindikator bereitzustellen, dessen Referenzkeime ausgewählt sind aus der Familie der Enterokokken und/oder Staphylokokken. Enterokokken sind bevorzugt grampositive, Katalase-negative und aerotolerante, anaerobe Bakterien. Die kugelförmigen (kokkoiden) Bakterien sind in Paaren oder kurzen Ketten angeordnet. Enterokokken sind bevorzugt folgende Arten: Enterococcus avium, Enterococcus casseliflavus, Enterococcus durans, Enterococcus faecalis, Enterococcus faecium, Enterococcus gallinarum, Enterococcus hirae, Enterococcus raffinosus. Staphylokokken sind bevorzugt rundliche, weintraubenähnlich angeordnete, nicht sporenbildende grampositive Bakterien ohne aktive Bewegung aus der Gruppe der Kokken. Es können sowohl koagulasepositive Staphylokokken als auch koagulasenegative Staphylokokken bevorzugt sein. Koagulasepositive Staphylokokken sind bevorzugt Staphylococcus aureus, Staphylococcus agnetis, Staphylococcus delphini, Staphylococcus hyicus, Staphylococcus intermedius, Staphylococcus lutrae, Staphylococcus pseudintermedius und/oder Staphylococcus schleiferi subsp. Coagulans. Koagulasenegative Staphylokokken sind bevorzugt Staphylococcus epidermidis, Staphylococcus haemolyticus, Staphylococcus lugdunensis und/oder Staphylococcus saprophyticus subsp. Saprophyticus.

Es ist besonders bevorzugt, Referenzkeime ausgewählt aus der Art Enterococcus faecium und/oder Staphylococcus aureus zu verwenden. Referenzkeimen aus dieser Art sind überraschend geeignet, sowohl für thermoresistente als auch nicht-thermoresistente Keime die Reduktion beim Desinfektionsvorgang gut abzubilden. Durch diese Referenzkeime kann eine mikrobiologische Prüfung von Waschmaschinen vorgenommen werden, durch welche der Desinfektionsvorgang auch für andere im klinischen Alltag vorkommende Keime auf überraschende Weise überprüft werden kann. Ebenso weisen bevorzugt Referenzkeime der Art Candida Albicans Eigenschaften auf, welche sie zu einer Art idealen Repräsentant auch anderer häufiger Keime macht. Daher ist die Aussagekraft bei einer Überprüfung mit diesen Keimen besonders hoch.

Insbesondere die Kombination Referenzkeime ausgewählt aus der Art Enterococcus faecium, Staphylococcus aureus und/oder Candida Albicans in Verbindung mit einer Polymermembran und einer Porengröße zwischen 0,1 µm und 2 µm und einer Keimdichte von bis zu 10⁷ hat sich als überraschend vorteilhafte Kombination herausgestellt.

Referenzkeime können auch bevorzugt ausgewählt sein aus der Gruppe Mycobacterium terrae, Mycobacterium avium, Bacillus atrophaeus, Aspergillus brasiliensis, Trychophyton rubrum, Candida albicans und/oder MS 2-Phage. Durch diese Auswahl von Referenzkeimen kann eine individuell an die zu prüfende Waschmaschine und ihre Umgebung angepasste mikrobiologische Prüfung erfolgen. Beispielsweise kann es nötig sein, dass für eine Prüfung von Waschmaschinen in tropischen Ländern, in denen die Infektionsgefahr von anderen Viren und/oder Bakterien ausgeht, andere Referenzkeime sinnvoll sind als in klimatisch gemäßigten Ländern.

Es ist bevorzugt, eine Keimdichte von mindestens 10⁶ aufzubringen. Solch ein Bioindikator kann besonders einfach und kostengünstig hergestellt werden. Es kann ebenso bevorzugt sein, eine Keimdichte von bis zu 10⁶ aufzubringen. Ein so hergestellter Bioindikator spart Arbeitsstufen und Zeit. Durch die bevorzugte Keimdichte kann ein individuell an den vorzunehmenden Desinfektionsvorgang angepasster Bioindikator bereitgestellt werden.

Es ist besonders bevorzugt, eine Keimdichte von mindestens 10⁷ aufzubringen. Ein solcher Bioindikator ist besonders robust gegenüber physischen Einflüssen. Es kann ebenso besonders bevorzugt sein, dass die Referenzkeime auf dem Träger eine Keimdichte von bis zu 10⁷ aufweisen. Mithilfe einer solchen Keimdichte lässt sich überraschend gut eine Keimreduktion aufgrund von Desinfektionsverfahren um mehrere Größenordnungen nachweisen.

Es kann ebenso bevorzugt sein, eine Keimdichte von mindestens 10⁸ aufzubringen. Dadurch kann eine zuverlässigere Prüfung als im Stand der Technik durchgeführt werden. Es kann auch bevorzugt sein, eine Keimdichte von bis zu 10⁸ aufzutragen. Dies hat eine überraschende Qualitätshebung der Prüfung zur Folge.

Es ist insbesondere bevorzugt, eine Keimdichte von bis zu 10⁷ aufzubringen. Es war überraschend, dass mit so einer Keimdichte eine besonders zuverlässige Prüfung vorgenommen werden konnte.

Insbesondere die Kombination Referenzkeime ausgewählt aus der Art Enterococcus faecium, Staphylococcus aureus und/oder Candida Albicans in Verbindung mit einer Polymermembran und einer Porengröße zwischen 0,1 µm und 2 µm und einer Keimdichte von bis zu 10⁷ hat sich als überraschend vorteilhafte Kombination herausgestellt. Durch den hohen Durchfluss der Desinfektionsflüssigkeit durch den Träger mit den Keimen bei dieser Polymermembran mit der bevorzugten Porengröße und die gleichzeitig besonders gute Filterung der bevorzugten Keime bei dieser Membran hat sich die bevorzugte Keimdichte als ausreichend für eine extrem sichere und statistisch relevante Überprüfung herausgestellt, da zum einen sichergestellt ist, dass eine Keimreduktion nicht durch Auswaschen der Keime ausgelöst wird. Zum anderen kann aber eine Desinfektion der Keime aufgrund des hohen Durchflusses mit Desinfektionsmittel der Flüssigkeit mit hoher Sicherheit erfolgen, so dass eine fehlgeschlagene Abtötung der Keime mit großer Sicherheit kein falscher Alarm ist, sondern auf eine Fehlfunktion der Waschmaschine hindeutet.

Typischerweise soll eine Keimreduktion um bevorzugt mindestens drei Größenordnungen (Faktor 10³) oder Log-Stufen erreicht werden. Solch eine Keimreduktion lässt sich besonders zuverlässig nachweisen. Es ist besonders bevorzugt, eine Keimreduktion um mindestens vier Größenordnungen (Faktor 10⁴) oder Log-Stufen zu erzielen. Die für diese Reduktion verfügbaren Nachweisverfahren sparen Zeit. Es ist ganz besonders bevorzugt, eine Keimreduktion um mindestens fünf Größenordnungen (Faktor 10⁵) oder Log-Stufen zu erreichen. Dies hat eine Beseitigung von Fehlern beim Nachweis zur Folge. In manchen Fällen ist es bevorzugt, das eine Keimreduktion von mindestens sechs Größenordnungen (Faktor 10⁶) oder Log-Stufen erreicht wird. So kann ein verbesserter Nachweis erbracht werden. Bei der bevorzugten Keimdichte von bis zu 10⁷ lässt sich vorgenannte Keimreduktion ohne großen Aufwand nachweisen. Zum Nachweis der Keimreduktion können überraschenderweise gängige Verfahren, wie beispielsweise bevorzugt die Gram-Färbung, lichtmikroskopische Untersuchungen, aber auch das Anwachsen einer Kultur auf einem Nährboden, verwendet werden.

Bevorzugt ist die Keimdichte größer als 10⁶, jedoch kleiner als 10⁷. Z. B. kann die Keimdichte 5 x 10⁶ betragen. Dies stellt einen technischen Fortschritt dar, da eine Verbilligung, Ersparnis an Zeit und Reduktion an Material gebraucht wird.

Es kann bevorzugt sein, dass die Membran Poren eine Größe von > 0,1 µm zu verwenden. Diese sind besonders einfach und kostengünstig herzustellen. Es können auch Poren einer Größe von < 0,1 µm bevorzugt zum Einsatz kommen. Diese haben eine verbesserte Filterfunktion gegenüber manchen Keimen zur Folge. Es kann ebenfalls bevorzugt sein, Poren einer Größe von höchstens 2 nm zu verwenden. Diese sind für eine besonders zuverlässige Durchführung der Prüfung geeignet.

In einer bevorzugten Ausführungsform weisen die Poren der Membran eine Größe < 2 µm auf. Diese haben eine überraschende Qualitätshebung des Prüfverfahrens zur Folge. Es ist dabei besonders bevorzugt, dass die Poren eine Größe von < 1 µm haben, denn so kann die Effektivität des Prüfverfahrens gesteigert werden. Es ist insbesondere bevorzugt, dass die Poren eine Größe von < 0,6 µm aufweisen. So können Fehler beim Prüfverfahren beseitigt werden.

Eine bevorzugte Porengröße beträgt somit zwischen 0,1 µm und 1 µm, bevorzugt zwischen 0,1µm und 0,6 µm. Somit kann die Sicherheit der Überprüfung und eine Vermeidung des Austretens der Keime weiter erhöht werden, insbesondere in Kombination mit einer Polymermembran, Referenzkeimen ausgewählt aus der Art Enterococcus faecium, Staphylococcus aureus und/oder Candida Albicans und einer Keimdichte von bis zu 10⁷.

Auch Kombinationen bevorzugter Parameterbereiche genannter Porengrößen können bevorzugt sein. So wird eine besonders hohe Flexibilität bei der Anpassung des Bioindikators an die jeweilige Anwendung erreicht, die technischen Möglichkeiten werden vermehrt.

Es war völlig überraschend, dass durch die bevorzugte Porengröße ein Bioindikator bereitgestellt werden konnte, welcher die erfindungsgemäße Aufgabe auf einfache und zuverlässige Weise löst. Insbesondere kann durch die bevorzugte Porengröße auf einfache Weise sichergestellt werden, dass keine der bevorzugten Referenzkeime vom Inneren der Membran nach außen gelangt.

In einer bevorzugten Ausführungsform beträgt die Porengröße 2 - 10 µm, bevorzugt 2 - 6µm und insbesondere 2 - 4 µm. es war überraschend, dass mit solch großen Poren die Keime gefiltert werden können. Gleichzeitig ist der Durchfluss höher, die Leistung wird gesteigert, bei gleichzeitiger Verbilligung durch Senkung der Materialkosten.

Bevorzugt können Membran Polymermembranen oder keramische Membran sein. Die Membranmaterialien können bevorzugt folgende Materialien oder Materialgruppen umfassen: Polysulfone, Polyethersulfon, Silikone, Polyamide, Polyamidimid, Polyamid Harnstoff, Polycarbonate, Zeolithe, Polyacrylnitril, Polyethylen, Polypropylen, Polytetrafluorethylen, Polyvinylidenfluorid, Polyvinylchlorid und/oder Polypiperazinamid. Diese Materialien eignen sich für eine besonders kostengünstige Herstellung. Ebenso erhöhen diese Materialien überraschenderweise den Filtereffekt. Dadurch kann bezüglich der Filterfunktion ein synergistischer Effekt erzielt werden, da die Materialien selber, unabhängig von der Membran, die Filterfunktion verbessern.

Die Membran können ebenfalls bevorzugt Cellulose, Celluloseester und/oder regenerierter Cellulose umfassen. So können besonders einfach auch recycelte Materialien zum Einsatz kommen und nachhaltig Ressourcen und Kosten gespart werden.

Es kann auch bevorzugt sein, dass die Membran Keramik und/oder Edelstahl umfasst. Mit diesen Materialien kann eine Membran mit einer besonders leistungsstarken Filterfunktion bereitgestellt werden.

Weiterhin kann bevorzugt sein, dass die Membran Silber und/oder Silizium umfasst. Durch diese Materialien kann die Membran verbessert werden.

Durch die bevorzugten Materialien zur Membranherstellung kann eine Membran bereitgestellt werden, die sowohl zur Lösung der erfindungsgemäßen Aufgabe beiträgt, als auch sehr einfach herzustellen ist. Insbesondere in Kombination mit der bevorzugten Porengröße ergeben sich synergistische Effekte, die auf überraschende Weise dazu beitragen, dass kein Referenzkeim vom Inneren der Membran nach außen gelangen kann. Es war völlig überraschend, dass durch die Kombination von bevorzugtem Material und bevorzugter Porengröße diese Filterfunktion der Membran sogar funktionieren kann, wenn die Referenzkeime kleiner sind als die Porengröße.

Es ist bevorzugt, dass der Träger eine flächige Form aufweist. Unter flächig ist bevorzugt zu verstehen, dass das Element in zwei Dimensionen eine größere Ausdehnung als in einer dritten Dimension aufweist. Die Träger können bevorzugt im Wesentliche rund oder eckig sein. Es kann bevorzugt sein, dass die Träger dreieckig, viereckig, quadratisch oder rechteckig sind, aber auch Fünfecke oder Sechsecke können bevorzugt sein. Es ist bevorzugt, dass die Form im Wesentlichen ein Polygon ist. Durch die Anpassung der Form an die Anwendung kann ein besonders vorteilhaftes Prüfverfahren durchgeführt werden.

Durch die bevorzugte, flächige Form kann auf überraschende Weise ein Bioindikator zur Verfügung gestellt werden, welcher den Waschvorgang bei der mikrobiologischen Prüfung von Waschmaschinen nicht stört. Auch bei vollen Waschmaschinen wird durch die Beschickung der Waschmaschine mit dem Bioindikator keine Beeinträchtigung des Waschvorgangs hervorgerufen. Insbesondere in Kombination mit der bevorzugten, flexiblen Ausführungsform ergibt sich ein synergistischer Effekt und die Anpassungsfähigkeit an den Beladungszustand der Waschmaschine kann in stärkerer Weise verbessert werden, als es durch die einzelne Betrachtung der Form und der Flexibilität des Indikators zu erwarten war. Somit ergibt sich bezüglich der Beladung der Waschmaschine mit Wäsche oder Waschgut sowie bezüglich der Güte des Waschvorgangs bei der Beschickung der Waschmaschine mit dem Bioindikator kein Unterschied zu einem normalen Waschvorgang ohne Bioindikator.

Eine Waschmaschine im Sinne der Erfindung umfasst bevorzugt alle Maschinen, die einen Desinfektionsvorgang durchführen bzw. nachweisen können. Hierbei kann es sich bspw. auch um Spül- oder andere -maschinen und -vorrichtungen handeln.

Es ist bevorzugt, dass das Trägermaterial textiles Material oder textile Materialien umfasst. Es können bevorzugt Naturfasern wie Steinwolle, Baumwollfasern, Flachsfasern, Hanffasern oder tierische Fasern wie z. B. Wolle, Seide oder Fellhaare zum Einsatz kommen. Diese weisen sich durch eine hohe Zuverlässigkeit sowie geringe Herstellungskosten aus. Außerdem können diese Trägermaterialien überraschenderweise die Filterfunktion der Membran verbessern. Es kann aber auch bevorzugt sein, dass Chemiefasern wie Viskose, Lyocell, Gummi oder aus synthetischen Polymeren wie z. B. Polyacrylnitril, Polypropylen, Polyester, Polyamid oder Polyurethan zum Einsatz kommen. Diese lassen sich besonders leicht verarbeiten und sparen Arbeitsstufen bei der Herstellung ein. Es können auch anorganische Fasern wie z. B. Keramik-, Glas- oder Metallfasern verwendet werden. Diese zeichnen sich durch eine besondere Effektivität als Trägersubstanz aus. Es kann auch bevorzugt sein, dass der Träger mit der Innenseite der Membran identisch ist oder zumindest aus dem gleichen Material besteht. So können Materialien eingespart und Kosten reduziert werden. Das Trägermaterial umfasst in diesem Fall bevorzugt folgende Materialien oder Materialgruppen: Polysulfone, Polyethersulfon Cellulose, Celluloseester, Regenerierte Cellulose, Silikone, Polyamide, Polyamidimid, Polyamid Harnstoff, Polycarbonate, Keramik, Edelstahl, Silber, Silizium, Zeolithe, Polyacrylnitril, Polyethylen, Polypropylen, Polytetrafluorethylen, Polyvinylidenfluorid, Polyvinylchlorid und/oder Polypiperazinamid.

Durch diese bevorzugte Ausführungsform des Trägers ergibt sich eine hohe Flexibilität bezüglich der Herstellung des Bioindikators. Besonders die bevorzugte Variante, bei der der Träger aus Naturfasern, wie beispielsweise Baumwolle besteht, führt zu einem besonders einfachen, robusten und kostengünstig herstellbaren Bioindikator.

Es ist bevorzugt, dass der Bioindikator ein Baumwollläppchen umfasst, insbesondere als Träger, auf dem eine kontaminierte Nährlösung (insbesondere mit den Referenzkeimen) aufgetragen wird. Ein solcher Bioindikator mit einem solchen bevorzugten Träger ist besonders einfach herzustellen, dabei überraschend robust.

In einem weiteren Aspekt betrifft die Erfindung also auch einen Kit, welches 2 bis 16 Bioindikatoren zur mikrobiologischen Überprüfung von Waschmaschinen aufweist. Diese Anzahl von Bioindikatoren verbessert die mikrobiologische Überprüfung. Es ist bevorzugt, dass das Kit 2 bzw. 4 bis 16 Bioindikatoren aufweist. Es ist stärker bevorzugt, dass das Kit 7 bis 12 Bioindikatoren aufweist. Bei einer solchen Anzahl kann die Qualität der Überprüfung gehoben werden. Es ist dabei besonders bevorzugt, dass das Kit 8 bis 11 Bioindikatoren aufweist. Eine solche Anzahl von Bioindikatoren erhöht die Effektivität. Insbesondere bevorzugt ist ein Kit, welches 10 Bioindikatoren zur mikrobiologischen Überprüfung von Waschmaschinen aufweist. Mit einem solchen Kit kann eine besonders zuverlässige Überprüfung durchgeführt werden. Das Kit umfasstdabei bevorzugt einen Anwendungsbogen, einen Begleitbogen und/oder mindestens einen Versandbeutel. Anwendungsbogen beschreibt bevorzugt eine Handlungsanweisung zur Durchführung des Prüfverfahrens. Begleitbogen beschreibt bevorzugt ein von der prüfenden Person auszufüllendes Formular zur Qualitätssicherung, in welches bevorzugt beispielsweise das Datum, Daten zur Zuordnung der geprüften Waschmaschine wie beispielsweise Standort, der Name und die Unterschrift des Prüfers und/oder das durchgeführte Waschprogramm eingetragen werden. Versandbeutel bezeichnet bevorzugt einen Beutel, der die sichere Versendung des Kits auf postalischem Wege ermöglicht. Dabei ist unter sicher zu verstehen, dass das Kit zerstörungsfrei und ohne wesentlichen Einfluss auf die Keimdichte verbleibt. In dieser bevorzugten Ausführungsform umfassend 4 bis 16, bevorzugt 7 bis 12, besonders bevorzugt 8 bis 11, insbesondere 10 Bioindikatoren, kann auf besonders zuverlässige Weise geprüft werden, ob ein Desinfektionsverfahren den Sicherheitsstandards genügt. Sollte ausnahmsweise einer oder gar mehrere der Bioindikatoren nicht ordnungsgemäß funktionieren, zum Beispiel, weil durch einen Fehler bei der Präparierung nicht die richtige Keimdichte von Referenzkeimen aufgebracht wurde oder weil die Poren der Membran verstopft wurden, können über die übrigen Bioindikatoren trotzdem zuverlässige Aussagen getroffen werden. Des Weiteren können über die Verwendung verschiedener Referenzkeime, beispielsweise von 5 Bioindikatoren mit den Referenzkeimen der Gruppe Enterococcus faecium und fünf Bioindikatoren mit den Referenzkeimen der Gruppe Staphylococcus aureus bessere Aussagen über den Erfolg des Desinfektionsverfahrens für verschiedenste Keime gemacht werden. Durch die bevorzugte Ausführungsform umfassend ebenfalls bevorzugt einen Anwendungsbogen, einen Begleitbogen und/oder mindestens einen Versandbeutel wird die Anwendung auch für nicht fachmännische Benutzer erleichtert. Insbesondere können die gewöhnlicherweise mit dem Wasch- oder Desinfektionsvorgang betrauten Fachkräfte ebenso die Benutzung der Bioindikatoren zur Prüfung der Desinfektionsleistungen der Waschmaschinen, mit denen die Desinfektion durchgeführt werden soll, z.B. Waschmaschinen durchführen. Durch den Bioindikator in der bevorzugten Ausführungsform sowie den zusätzlichen bevorzugten Anwendungs- und/oder Begleitbogen ist die Durchführung der mikrobiologischen Prüfung leicht und fehlerfrei handhabbar. Die eigentliche Überprüfung der Desinfektionsleistung kann von einem dritten Dienstleister oder vom Hersteller des Bioindikators vorgenommen werden. Dazu werden die Bioindikatoren einfach mittels des bevorzugt umfassten mindestens einen Versandbeutels eingesendet. Durch diese einfache Handhabung kann ein sehr zuverlässiges Prüfverfahren gewährleistet werden und eine versehentliche oder mutwillige falsche Durchführung des Prüfverfahrens wird vermieden. Durch die einfache Handhabung des Prüfverfahrens wird eine überraschend hohe Compliance, d. h. bevorzugt eine hohe Wahrscheinlichkeit der Einhaltung der bevorzugten Prüfabstände sowie Prüfabläufe, gewährleistet. Darüber hinaus können die Bioindikatoren eines solchen Kits, welche geeignete, hier beschriebene Merkmale aufweisen, den Vorschriften bzw. Richtlinien zahlreicher Länder, wie bspw. Deutschland oder Österreich, bezüglich der hier relevanten Prüfverfahren genügen. So kann eine Verbesserung des Prüfverfahrens durch die Benutzung eines solchen Kits erzielt werden, da der Benutzer gleichzeitig die eigene Qualitätskontrolle sowie die ggf. staatlich vorgeschriebene Überprüfung durchführen kann. Kosten und Arbeitsstufen können so gespart werden. Da die Vorschriften und Richtlinien sich an langjährigen Erfahrungen der Behörden orientieren und somit die Praxis der hier relevanten Überprüfungen abbilden, kann die Sicherheit zusätzlich erhöht werden. Des Weiteren wird durch die hier vorgestellten Bioindikatoren zur Erfüllung der Vorschriften und/oder Richtlinien ein neuer Weg beschritten.

In einer bevorzugten Ausführungsform des Kits sind 7 bis 12 Bioindikatoren umfasst, wobei deren Membran eine Polymermembran ist mit einer bevorzugten Porengröße zwischen 0,1 µm und 2 µm, besonders bevorzugt zwischen 0, 1 µm und 1 µm und insbesondere zwischen 0,1µm und 0,6 µm, wobei die Referenzkeimen ausgewählt sind aus der Art Enterococcus faecium, Staphylococcus aureus und/oder Candida Albicans und die jeweilige Keimdichte pro Indikator bis zu 10⁷ beträgt. Ein solches Kit kann überraschend verbesserte statistische Aussagen über die Desinfektionsleistung einer Waschmaschine treffen, welche aus dem Stand der Technik nicht bekannt sind und falsche Aussagen der Überprüfung so gut wie ausschließen. Die Vorteile, die sich bereits bei einzelnen Bioindikatoren mit den bevorzugten Eigenschaften bemerkbar machen, führen so, insbesondere wenn verschiedene Keime pro Indikator in einem Kit verwendet werden, zu überraschend verbesserten statistischen Aussagen der Überprüfung. Je nach Porengröße kann dabei entweder die Sicherheit vor Austritt der Keime verbessert oder das Auftreten eines Fehlalarms verringert werden.

In einer bevorzugten Ausführungsform umfasst das Kit 2 Bioindikatoren, wobei ein 1 Bioindikator Referenzkeime einer Art, bevorzugt Enterococcus faecium aufweist und der zweite Bioindikator Referenzkeime einer zweiten Art, bevorzugt Candida Albicans und/oder Staphylococcus aureus aufweist. Ein solcher Bioindikator ist besonders einfach herzustellen und zu handhaben. Durch die Kompaktheit wird ein Waschgut besonders wenig geschädigt. Durch die Verwendung der zwei Referenzkeime kann trotz der Kompaktheit eine umfassende Prüfung der Desinfektionsleistung bezüglich mehrerer Keime vorgenommen werden. Es war überraschend, dass insbesondere die Kombination aus Enterococcus faecium und Candida Albicans bzw. Staphylococcus aureus eine besonders umfassende, sichere und statistisch aussagekräftige Überprüfung von Waschvorgängen zulässt.

In einer weiteren bevorzugten Ausführungsform umfasst das Kit 4 Bioindikatoren. Es hat sich überraschenderweise gezeigt, dass 4 Bioindikatoren für eine sichere und statistisch relevante Überprüfung der Desinfektionsleistung besonders geeignet sind.

Es kann bevorzugt sein, dass das Kit des Weiteren mindestens ein Baumwollsäckchen umfasst, in welches die Bioindikatoren während des Waschvorgangs eingebracht werden können. Dies kann zu einer zusätzlichen Verbesserung der Filterfunktion der Membran beitragen und somit die Sicherheit und Zuverlässigkeit des Verfahrens erhöhen. Darüber hinaus können so die Waschmaschine und das Waschgut geschont und somit langfristig Kosten gespart werden.

In einer weiteren bevorzugten Ausführungsform umfasst das Kit 10 Bioindikatoren, wobei 5 Bioindikatoren Referenzkeime einer Art, bevorzugt Enterococcus faecium in unterschiedlichen Log-Stufen aufweisen und 5 Bioindikatoren Referenzkeime einer zweiten Art, bevorzugt Candida Albicans und/oder Staphylococcus aureus in unterschiedlichen Log-Stufen aufweisen. Es wurde überraschend festgestellt, dass in dieser Ausführungsform der Erfindung eine besonders hohe Sicherheit bei der Überprüfung der Desinfektionsleistung bei gleichzeitig enormer statistischer Relevanz resultiert. Die Kombination der Keime ist für eine umfassende Aussage über die Desinfektionsleistung verschiedenster, auch anderer Keime besonders geeignet. Die bevorzugt verwendeten Keimkombinationen weisen vorteilhafterweise Eigenschaften auf, welche mit einer gewissen Universalität auch andere, häufige Keime repräsentieren. Durch die Verwendung verschiedener Log-Stufen kann die statistische Aussagekraft der Überprüfung enorm erhöht werden. Verschiedene Log-stufen bezeichnen dabei bevorzugt Referenzkeimanzahlen bzw. -dichten, welche sich in der Zehnerpotenz um mindestens 1 unterscheiden.

Eine vorteilhafte Ausführungsform betrifft ein Kit, wobei das Kit 2 bis 8, bevorzugt 3 bis 6, besonders bevorzugt 4 bis 6, insbesondere 5 Bioindikatoren Referenzkeime aufweisen aus der Gruppe Enterococcus faecium und 2 bis 8, bevorzugt 3 bis 6, besonders bevorzugt 4 bis 6, insbesondere 5 Bioindikatoren Referenzkeime aufweisen aus der Gruppe Staphylococcus aureus und wobei die Anzahl der Bioindikatoren mit Referenzkeimen aus der einen Gruppe bevorzugt der Anzahl der Bioindikatoren aus der anderen Gruppe entspricht.

Es ist also in dieser Ausführungsform bevorzugt, wenn das Kit jeweils die gleiche Anzahl an Bioindikatoren mit Referenzkeimen aus der Gruppe Enterococcus faecium und an Bioindikatoren mit Referenzkeimen aus der Gruppe Staphylococcus aureus aufweist. Durch diese bevorzugte Ausführungsformen kann auf besonders zuverlässige Weise der Erfolg von Desinfektionsverfahren überprüft werden. Es ist dabei bevorzugt, dass das Kit für jeden Referenzkeim 1 bis 8 Bioindikatoren, insbesondere 2 bis 8 Bioindikatoren, also insgesamt 2 bis 16 Bioindikatoren, bevorzugt 4- 16 Bioindikatoren aufweist. Ein solches Kit ist besonders robust. Es ist dabei weiterhin bevorzugt, dass das Kit 3 bis 6 Bioindikatoren für jeden Referenzkeim, also insgesamt 6 bis 12 Bioindikatoren umfasst. So kann eine Erhöhung der Effektivität des Kits erreicht werden. Besonders bevorzugt ist, dass das Kit 4 bis 6 Bioindikatoren für jeden Referenzkeim, also insgesamt 8 bis 12 Bioindikatoren aufweist. Solch ein Kit trägt zur Fehlerbeseitigung bei der Überprüfung bei. Insbesondere ist bevorzugt, dass das Kit 5 Bioindikatoren für jeden Referenzkeim, also insgesamt 10 Bioindikatoren, aufweist. Somit ist es auch möglich, ein Kit mit bspw. 10 Bioindikatoren einzusetzen mit jeweils der gleichen Anzahl an Bioindikatoren mit Referenzkeimen aus der Gruppe Enterococcus faecium und Referenzkeimen aus der Gruppe Staphylococcus aureus. Überraschenderweise hat sich herausgestellt, dass ein solches Kit zu einer besonders zuverlässigen Überprüfung von Waschmaschinen führt. Darüber hinaus kann es besonders kostengünstig hergestellt werden. Die Produktion eines solchen Kits kann besonders gut rationalisiert werden. Insbesondere sprechen Referenzkeimen aus der Gruppe Enterococcus faecium und Staphylococcus aureus auf unterschiedliche Aspekte eines Desinfektionsverfahrens an. Beispielsweise besitzt Enterococcus faecium eine recht hohe Thermoresistenz und kann daher insbesondere als Repräsentant thermoresistenter Keime im Prüfverfahren angesehen werden. Beide Gruppen von Keimen decken in ihrem Ansprechverhalten bei einem Desinfektionsverfahren das Verhalten einer Vielzahl von Keimen ab. Es war völlig überraschend, dass sich durch die bevorzugte Verwendung von je fünf Bioindikatoren mit Referenzkeimen aus der Gruppe Enterococcus faecium bzw. Staphylococcus aureus eine äußert effektive, zuverlässige und kostengünstige Überprüfung von Desinfektionsverfahren für Waschmaschinen realisieren ließ.

Die Kombination verschiedenster Anzahlen von Indikatoren und/oder Referenzkeimen und/oder Keimdichten ist auf Grund der Verarbeitung der Membran und der Bioindikatoren besonders flexibel und bevorzugt jedem Kundenwunsch anzupassen.

Es ist vorteilhaft, wenn das Kit an der Außenfläche klar erkenntlich eine eindeutig zuordenbare Nummer trägt, die beispielsweise bevorzugt durch einen Stempel aufgebracht wurde. Durch die Nummer kann das Kit schnell und eindeutig identifiziert werden. Mit dieser Nummer können bevorzugt beispielsweise Daten wie die zu überprüfende bzw. überprüfte Desinfektionsstelle, die verwendeten Referenzkeime sowie ihre Dichte, das Herstellungsdatum des Bioindikators, etc., verknüpft sein.

Durch diese bevorzugte Ausführungsform kann auf überraschende Weise eine fehlerhafte Zuordnung des Kits vermieden werden. Die Nummer kann bevorzugt als redundantes Identifikationsmerkmal neben dem ausgefüllten Begleitbogen angesehen werden. So kann vermieden werden, dass ein aus einer mikrobiologischen Prüfung einer Waschmaschine mithilfe des Kits resultierendes Prüfergebnis einer falschen Waschmaschine zugeordnet wird. Solch eine falsche Zuordnung könnte hohe Kosten und ein hohes gesundheitliches Risiko durch Infektionen aufgrund eines nicht funktionierenden, nicht richtig identifizierten Desinfektionsvorgangs zur Folge haben.

Es ist bevorzugt, dass die einzelnen Bioindikatoren des Kits zusammengefasst in einem flächigen Element vorliegen. Ein solches Kit trägt zur Materialersparnis bei. Die Form des Elements ist im Wesentlichen bevorzugt rechteckig. Ein solches Element ist überraschenderweise besonders gut geeignet, einen reibungslosen Waschgang während der Prüfung zu gewährleisten. Das Element ist bevorzugt sichtbar unterteilt in die einzelnen Bioindikatoren. Das trägt zur Sicherheit und Zuverlässigkeit des Prüfverfahrens bei, insbesondere können Fehler bei der Herstellung der Bioindikatoren leichter entdeckt werden. Es ist bevorzugt, dass das Element mechanisch stabil, dennoch flexibel ist. Ein solches Element schont die zu überprüfende Waschmaschine sowie das Waschgut und trägt so zur Reduktion von Kosten bei. Bevorzugt ist an einer Randfläche des Elements die Zuordnungsnummer sichtbar. Das erhöht zusätzlich die Sicherheit und Zuverlässigkeit, Fehler können besser behoben werden. Sowohl in der bevorzugten Ausführungsform, bei der der Träger die Innenseite der Membran selber ist, als auch bei der bevorzugten Ausführungsform, wo der Träger textiles Material oder textile Materialien aufweist, kann es bevorzugt sein, dass die einzelnen Bioindikatoren durch Schweißnähte der Membran voneinander getrennt sind. So kann das Überprüfungsverfahren verbessert werden, es kann nicht zu einer gegenseitigen Kontamination der Bioindikatoren kommen. Es kann bevorzugt sein, an einigen Stellen doppelte Schweißnähte zwischen den Bioindikatoren einzufügen, zwischen denen kleine, nicht verschweißte, flächige Elemente entstehen. Diese doppelten Schweißnähte sowie die kleinen, flächigen Elemente können zur klaren Kennzeichnung der verschiedenen Bioindikatoren, beispielsweise zur Unterscheidung der von Ihnen getragenen Referenzkeime, verwendet werden. Dies erhöht die Zuverlässigkeit insbesondere bei der Kontrolle der Keimreduktion. Es kann auch bevorzugt sein, dass die kleinen, flächigen Elemente nicht Träger von Referenzkeimen sind und somit zur Kontrolle der Funktionsfähigkeit des Bioindikators herangezogen werden können. So können Fehler behoben werden. Es kann zur Kontrolle der Funktionsfähigkeit auch bevorzugt sein, dass sie Referenzkeime tragen, aber nicht von einer semipermeablen Membran, sondern einer undurchlässigen Membran umgeben sind. So kann die Zuverlässigkeit des Verfahrensüberprüft und erhöht werden. Es können auch beide Funktionalitäten der kleinen, flächigen Elemente zugleich bevorzugt Anwendung finden, indem beispielsweise unterschiedliche Elemente unterschiedlich genutzt werden. Dies hat einen synergistischen Effekt bezüglich der Sicherheit und Zuverlässigkeit des Verfahrens sowie der Beseitigung von Fehlern zur Folge, da sich diese Aspekte in einem Maße verbessern, welches über das Maß hinausgeht, welches die einzelnen Anwendungen der kleinen flächigen Elemente für sich genommen erwarten lassen.

In einem weiteren Aspekt betrifft die Erfindung auch ein Verfahren zur mikrobiologischen Prüfung von Waschmaschinen, umfassend bevorzugt folgende Schritte: die Entnahme mindestens eines Bioindikators aus einem Versandbeutel, die Beschickung einer zu prüfenden Waschmaschine mit dem mindestens einen Bioindikator, bevorzugt gemeinsam mit einem zu desinfizierenden und/oder zu waschenden Gut, das Ausfüllen des Begleitbogens, die Entnahme und vorzugsweise die Trocknung des mindestens einen Bioindikators aus der Waschmaschine nach dem Desinfektionsvorgang, und schließlich das Einlegen des mindestens einen Bioindikators und des Begleitbogens in einen Versandbeutel zum Zwecke seiner Rücksendung sowie der Rücksendung des Bioindikators und des Begleitbogens im Versandbeutel. Das bevorzugte Kit wird in einem Versandbeutel, welcher zum sicheren Versenden geeignet ist, an die Prüfstelle bevorzugt postalisch zugestellt. Der Versandbeutel kann dabei bevorzugt auch zur sicheren Lagerung des Kits verwendet werden. Vor der Prüfung kann das Kit bevorzugt dem Versandbeutel entnommen werden. Es kann bevorzugt sein, dass ein Begleitbogen sowie ein bevorzugt vorhandener, zweiter Versandbeutel zur Rücksendung ebenfalls dem Versandbeutel beiliegen und entnommen werden kann. Zur Prüfung wird die zu prüfende Maschine, bspw. die Spül- oder Waschmaschine, mit dem Kit beschickt. Dabei kann es bevorzugt sein, dass Kit einzeln oder gemeinsam mit Wäsche der Waschmaschine zugefügt wird. Im zweiten Fall kann es bevorzugt sein, das Kit lose oder mit der Wäsche wirkverbunden zuzufügen. Unter wirkverbunden kann bevorzugt beispielsweise die Verbindung des Kits mit der Wäsche mittels eines Kabelbinders, aber auch die Verbindung durch Einwickeln in die Wäsche oder durch Einstecken in einer in der Wäsche vorhandenen Tasche gemeint sein. Nach der Einstellung des zu prüfenden Waschprogramms, der Zugabe des bevorzugt zu verwendenden Waschmittels und dem Starten des Waschvorgangs kann bevorzugt das Ausfüllen des Begleitbogens durch die prüfende Person vorgenommen werden. Dabei sind bevorzugt für die Identifizierung der geprüften Waschmaschine relevante Daten, das Datum, der Name und die Unterschrift der prüfenden Person, das gewählte Waschprogramm und andere eventuell relevante Informationen und Anmerkungen einzutragen. Das Ausfüllen des Begleitbogens kann natürlich auch zu einem späteren Zeitpunkt vor der Rücksendung vorgenommen werden. Nach Beendigung des Waschvorgangs wird der Bioindikator der Waschmaschine entnommen, bevorzugt getrocknet, und dann gemeinsam mit dem Begleitbogen in einen Versandbeutel eingelegt. Dabei kann es sich bevorzugt um einen neuen, ebenfalls mitgelieferten Versandbeutel, aber auch um den gleichen Versandbeutel wie der zur vorherigen Zustellung verwendete handeln. Bevorzugt trägt der Versandbeutel bereits die Adresse des Dienstleisters bzw. der Prüfstelle zur Überprüfung der Keimreduktion. Dann kann der Versandbeutel samt Inhalt versendet werden. Insbesondere die Kompaktheit und bevorzugte Flächigkeit des Kits bzw. Indikators sowie dessen Sicherheit machen das Kit bzw. den Bioindikator für ein Versenden geeignet.

Es kann bevorzugt sein, dass bei der Beschickung der zu prüfenden Waschmaschine mit mindestens einen Bioindikator dieser vorher in ein Baumwollsäckchen eingebracht wird, um die Sicherheit und Zuverlässigkeit des Verfahrens zu erhöhen und die Kosten zu senken.

Der mindestens eine Bioindikator dieses Verfahrens kann vorteilhafterweise auch als Element umfassend mehrere Bioindikatoren vorliegen und/oder die in einem Kit enthaltenen Bioindikatoren umfassen.

Durch diese bevorzugte Ausführungsform kann ein überraschend sicheres, zuverlässiges, effizientes, kostengünstiges und einfach durchzuführendes Verfahren zur mikrobiologischen Überprüfung von z. B. Waschmaschinen bereitgestellt werden. Es war völlig überraschend, dass durch die vorangestellten Verfahrensschritte eine äußerst hohe Compliance bei der Durchführung der Prüfung der Desinfektionsleistung von Waschmaschinen gewährleistet werden konnte. Auch nicht sachkundiges Personal, welches zum Teil unter hohem Zeitdruck arbeitet, und für welches die Überprüfung der Desinfektionsleistung eine lästige Zusatzarbeit bedeutet, wurde durch vorangestelltes Verfahren in die Position gebracht, fehlerfrei und ohne große Zeitverluste ein Verfahren zur mikrobiologischen Überprüfung von Waschmaschinen durchzuführen. Darüber hinaus kann das Verfahren unter Verwendung geeigneter Bioindikatoren den Vorschriften bzw. Richtlinien zahlreicher Länder, wie bspw. Deutschland oder Österreich, bezüglich der relevanten Prüfverfahren genügen. So kann eine Verbess erung des Prüfverfahrens erzielt werden, da der Benutzer gleichzeitig die eigene Qualitätskontrolle sowie die ggf. staatlich vorgeschriebene Überprüfung durchführen kann. Kosten und Arbeitsstufen können so gespart werden. Da die Vorschriften und Richtlinien sich an langjährigen Erfahrungen der Behörden orientieren und somit die Praxis von den hier relevanten Überprüfungen abbilden, kann die Sicherheit zusätzlich erhöht werden. Des Weiteren wird durch die hier vorgestellten Bioindikatoren zur Erfüllung der Vorschriften und/oder Richtlinien ein neuer Weg beschritten.

In einem weiteren Aspekt betrifft die Erfindung die Verwendung des Bioindikators zur mikrobiologischen Prüfung von Waschmaschinen. Der Fachmann erkennt, dass die bevorzugten Ausführungsformen und Vorteile der Vorrichtung und des Verfahrens ebenfalls für die Verwendung gelten.

### Kurzbeschreibung der Abbildung

**Figur** 1 zeigt eine schematische Draufsicht einer bevorzugten Ausführungsform der Erfindung umfassend ein Kit, welches 10 Bioindikatoren zur mikrobiologischen Überprüfung von Waschmaschinen aufweist.
**Figur 2** zeigt eine Draufsicht eines einzelnen Bioindikators.
**Figur 3** zeigt einen Querschnitt eines einzelnen Bioindikators.

### Detaillierte Beschreibung der Abbildung

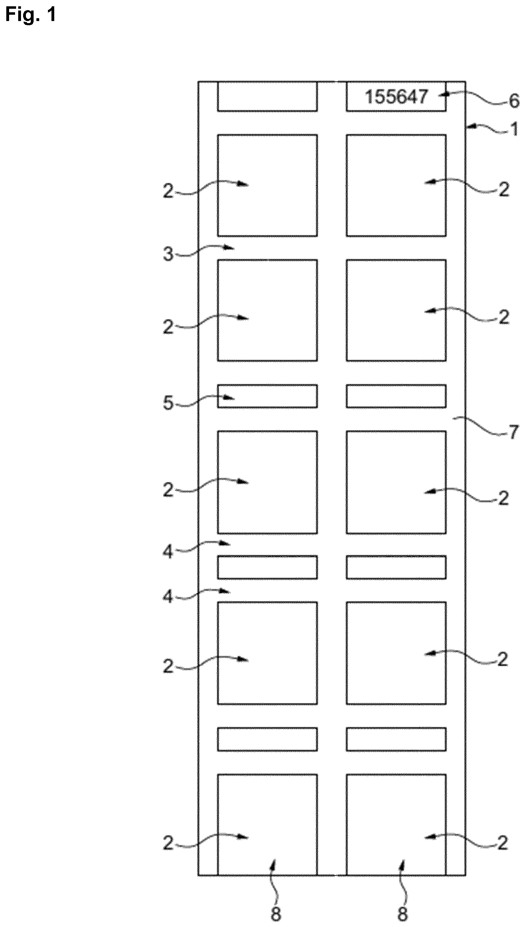

Figur 1 zeigt eine schematische Draufsicht einer bevorzugten Ausführungsform der Erfindung umfassend ein Kit **1,** welches 10 Bioindikatoren **2** zur mikrobiologischen Überprüfung von Waschmaschinen aufweist. Die Schweißnähte **3** und doppelten Schweißnähte **4** liegen zwischen den Bioindikatoren bzw. den kleinen, flächigen Elementen **5** sowie an den Außenkanten **7** des Kits **1** vor. In der gezeigten, bevorzugten Ausführungsform des Kits liegen die Bioindikatoren **2** paarweise nebeneinander vor. Fünf dieser Paare **8** sind in einem Kit übereinander gruppiert. Es kann bevorzugt sein, dass jeweils ein Bioindikator **2** des Paars **8** mit einem Referenzkeim der Gruppe Enterococcus faecium und der andere mit einem Referenzkeim der Gruppe Staphylococcus aureus versehen ist. Es kann bevorzugt sein, dass immer der in der Draufsicht linke Bioindikators des Paares **8** mit einem der beiden Referenzkeime versehen ist und immer der rechte Bioindikator mit dem anderen. Eine zusätzliche Strukturierung wie in der gezeigten, bevorzugten Ausführungsform des Kits **1** ist bevorzugt. Beispielsweise ist bevorzugt, die doppelten Schweißnähte **4** und die kleinen, flächigen Elemente **5** zwischen den (in der Draufsicht) unteren drei Paaren **8** der Bioindikatoren **2** anzuordnen. So kann bei dem Bioindikator gut zwischen oben und unten, wie sie in der vorliegenden Zeichnung definiert sind, unterschieden werden. Weiterhin ist in der bevorzugten Ausführungsform am oberen Rand, bevorzugt am oberen rechten Rand, eine eindeutig zuordenbare Nummer **6** gut lesbar angebracht.

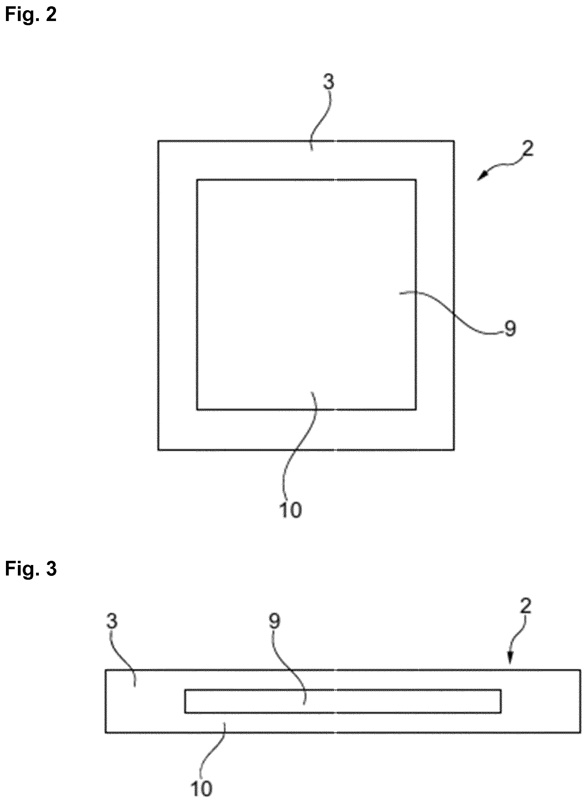

Figur 2 zeigt eine Draufsicht eines einzelnen Bioindikators **2,** wobei mittig der Träger **9** vorliegt und die Membran **10** außen durch eine Schweißnaht **3** um den Träger **9** herum geschlossen ist.

Figur 3 zeigt einen Querschnitt durch einen einzelnen Bioindikator **2,** wobei man sieht, dass der Träger **9** allseitig von der Membran **10** umschlossen ist. An den Seiten ist diese bevorzugt durch eine einfache Schweißnaht **3** umschlossen.

### Bezugszeichenliste:

- 1: Kit
- 2: Bioindikator
- 3: einfache Schweißnaht
- 4: doppelte Schweißnaht
- 5: flächige Elemente
- 6: eindeutig zuordenbare Nummer
- 7: Außenkanten
- 8: Paare von Bioindikatoren
- 9: Träger
- 10: Membran

## Patentansprüche

1. Bioindikator (2) zur mikrobiologischen Prüfung von Waschmaschinen, wobei
- der Bioindikator (2) einen flächigen Träger (9) umfasst,
- der Träger (9) Bakterien und/oder Pilze als Referenzkeime aufweist, wobei
- der Träger durch eine semipermeable Membran (10) umschlossen ist
**dadurch gekennzeichnet, dass**
die Membran (10) eine Polymermembran ist, Poren einer Größe zwischen 0,1 µm und 1 µm aufweist, für eine Desinfektionsflüssigkeit durchlässig ist und für die Referenzkeime undurchlässig ist und nach außen durch eine Schweißnaht um den Träger herum geschlossen ist, wobei die Polymermembran eine Dicke zwischen 0,005 mm und 1 mm aufweist, flexibel ist und eine mechanische Stabilität aufweist, welche einen Einsatz des Bioindikators ohne Schutzhülle bei einem Waschgang zur mikrobiologischen Prüfung der Waschmaschinen ermöglicht und wobei der Träger eine Dicke von weniger als 1 mm aufweist.

2. Bioindikator (2) nach Anspruch 1,
**dadurch gekennzeichnet, dass**
die Membran (10) hydrophob ist.

3. Bioindikator (2) nach einem oder mehreren der vorigen Ansprüche,
**dadurch gekennzeichnet, dass**
der Bioindikator (2) flächig ist.

4. Bioindikator (2) nach einem oder mehreren der vorigen Ansprüche,
**dadurch gekennzeichnet, dass**
der Träger (9) beidseitig für eine Desinfektionsflüssigkeit durchlässig ist.

5. Bioindikator (2) nach einem oder mehreren der vorigen Ansprüche,
**dadurch gekennzeichnet, dass**
die Referenzkeime ausgewählt sind aus der Gruppe Enterococcus faecium und/oder Staphylococcus aureus und/oder die Referenzkeime auf dem Träger (9) eine Keimdichte von bis zu 10⁷ aufweisen.

6. Bioindikator (2) nach einem oder mehreren der vorigen Ansprüche,
**dadurch gekennzeichnet, dass**
die Membran (10) Poren einer Größe von < 1 µm, bevorzugt < 0,6 µm aufweist.

7. Bioindikator (2) nach einem oder mehreren der vorigen Ansprüche,
**dadurch gekennzeichnet, dass**
die semipermeable Polymermembran ausgewählt ist aus der Gruppe umfassend Polysulfon, Polyethersulfon, Silikonen, Polyamid, Polyamidimid, Polyamid Harnstoff, Polycarbonat, Zeolithe, Polyacrylnitril, Polyethylen, Polypropylen, Polytetrafluorethylen, Polyvinylidenfluorid, Polyvinylchlorid und/oder Polypiperazinamid.

8. Kit (1), aufweisend 2 bis 16 Bioindikatoren (2) nach einem oder mehreren der vorigen Ansprüche, sowie einen Anwendungsbogen, einen Begleitbogen und/oder mindestens einen Versandbeutel, wobei die einzelnen Bioindikatoren (2) des Kits zusammengefasst in einem flächigen Element vorliegen.

9. Kit nach Anspruch 8
**dadurch gekennzeichnet, dass**
das Kit 2, 4 oder 10 Bioindikatoren (2) aufweist.

10. Kit (1) nach Anspruch 8 oder 9, umfassend zwei Bioindikatoren (2),
**dadurch gekennzeichnet, dass**
ein erster Bioindikator (2) Referenzkeime einer Art, bevorzugt Enterococcus faecium aufweist und ein zweite Bioindikator (2) Referenzkeime einer zweiten Art, bevorzugt Candida Albicans und/oder Staphylococcus aureus aufweist.

11. Kit (1) nach Anspruch 8, umfassend 10 Bioindikatoren (2),
**dadurch gekennzeichnet, dass**
5 Bioindikatoren (2) Referenzkeime einer Art, bevorzugt Enterococcus faecium in unterschiedlichen Log-Stufen aufweisen und 5 Bioindikatoren (2) Referenzkeime einer zweiten Art, bevorzugt Candida Albicans und/oder Staphylococcus aureus in unterschiedlichen Log-Stufen aufweisen.

12. Kit (1) nach Anspruch 8,
**dadurch gekennzeichnet, dass**
2 bis 8, bevorzugt 3 bis 6, besonders bevorzugt 4 bis 6, insbesondere 5 Bioindikatoren (2) Referenzkeime aufweisen aus der Gruppe Enterococcus faecium und 2 bis 8, bevorzugt 3 bis 6, besonders bevorzugt 4 bis 6, insbesondere 5 Bioindikatoren (2) Referenzkeime aufweisen aus der Gruppe Staphylococcus aureus und wobei die Anzahl der Bioindikatoren (2) mit Referenzkeimen aus der einen Gruppe der Anzahl der Bioindikatoren (2) aus der anderen Gruppe entspricht.

13. Verfahren zur mikrobiologischen Prüfung von Waschmaschinen, umfassend die folgenden Schritte
- Entnehmen mindestens eines Bioindikators (2) nach einem oder mehreren der Ansprüche 1 - 8 oder eines Kits (1) nach einem oder mehreren der vorherigen Ansprüche 9 - 13, aus einem Versandbeutel,
- Beschickung der zu prüfenden Waschmaschine mit dem mindestens einen Bioindikator (2) oder dem Kit (1) gemeinsam mit einem zu desinfizierenden und/oder zu waschenden Gut,
- Ausfüllen des Begleitbogens,
- Entnahme und bevorzugt Trocknung des mindestens einen Bioindikators (2) oder des Kits (1),
- Einlegen des mindestens einen Bioindikators (2) oder des Kits (1) und des Begleitbogens in einen Versandbeutel und Rücksendung des Bioindikators (2) oder des Kits (1) und des Begleitbogens.

14. Verwendung des Bioindikators (2) nach einem oder mehreren der Ansprüche 1 bis 7 und/oder eines Kits (1) nach einem oder mehreren der Ansprüche 8 bis 12 zur mikrobiologischen Prüfung von Waschmaschinen.

## Claims

1. Bioindicator (2) for microbiological testing of washing machines, wherein
- the bioindicator (2) comprises a flat carrier (9),
- the carrier (9) has bacteria and/or fungi as reference germs, wherein
- the carrier is enclosed by a semi-permeable membrane (10)
**characterized in that**
the membrane (10) is a polymer membrane, has pores of a size between 0.1 µm and 2 µm being permeable to a disinfectant liquid and impermeable to the reference germs and being sealed to the outside around the carrier by a weld, wherein the polymer membrane has a thickness between 0.005 to 1 mm, is flexible and has a mechanical stability allowing the bioindicator to be used without an additional protective cover in a washing machine for microbiological testing of the washing machine and wherein the carrier has a thickness of less than 1 mm.

2. Bioindicator (2) according to claim 1,
**characterized in that**
the membrane (10) is hydrophobic.

3. Bioindicator (2) according to one or more of the previous claims,
**characterized in that**
the bioindicator (2) is flat.

4. Bioindicator (2) according to one or more of the previous claims,
**characterized in that**
the carrier (9) is permeable on both sides for a disinfecting liquid.

5. Bioindicator (2) according to one or more of the previous claims,
**characterized in that**
the reference germs are selected from the group Enterococcus faecium and/or Staphylococcus aureus and/or the reference germs on the carrier (9) have a germ density of up to 10⁷.

6. Bioindicator (2) according to one or more of the previous claims,
**characterized in that**
the membrane (10) has pores of a size of < 1 µm, preferably < 0.6 µm.

7. Bioindicator (2) according to one or more of the previous claims,
**characterized in that**
the semipermeable polymer membrane is selected from the group comprising polysulfone, polyethersulfone, silicones, polyamide, polyamide imide, polyamide urea, polycarbonate, zeolites, polyacrylonitrile, polyethylene, polypropylene, polytetrafluoroethylene, polyvinylidene fluoride, polyvinyl chloride and/or polypiperazinamide.

8. Kit (1) comprising 2 to 16, bioindicators (2) according to one or more of the previous claims, and comprising an application sheet, an accompanying sheet and/or at least one mailing bag, wherein the individual bioindicators (2) of the kit are presently combined in a flat element.

9. Kit according to claim 8
**characterized in that**
the kit comprises 2, 4, or 10 bioindicators (2).

10. Kit (1) according to claim 8 or 9, comprising two bioindicators (2),
**characterized in that**
a first bioindicator (2) comprises reference germs of one species, preferably Enterococcus faecium, and the second bioindicator (2) comprises reference germs of a second species, preferably Candida Albicans and/or Staphylococcus aureus.

11. Kit (1) according to claim 8, comprising 10 bioindicators (2),
**characterized in that**
5 bioindicators (2) comprise reference germs of one species, preferably Enterococcus faecium, in different log levels and 5 bioindicators (2) comprise reference germs of a second species, preferably Candida Albicans and/or Staphylococcus aureus, in different log levels.

12. Kit (1) according to claim 8,
**characterized in that**
2 to 8, preferably 3 to 6, particularly preferably 4 to 6, in particular 5 bioindicators (2) comprise reference germs from the group Enterococcus faecium and 2 to 8, preferably 3 to 6, particularly preferably 4 to 6, in particular 5 bioindicators (2) comprise reference germs from the group Staphylococcus aureus and wherein the number of bioindicators (2) with reference germs from one group corresponds to the number of bioindicators (2) from the other group.

13. Method for the microbiological testing of washing machines, comprising the following steps
- Removal of at least one bioindicator (2) according to one or more of claims 1-8, or of a kit (1) according to one or more of the previous claims 9-13 from a mailing bag,
- Loading of the washing machine to be tested with the at least one bioindicator (2) or the kit (1) together with a good to be disinfected and/or washed,
- Fill in the accompanying sheet,
- Removal and preferably drying of the at least one bioindicator (2) or the kit (1),
- Insert at least one bioindicator (2) or the kit (1) and the accompanying sheet into a mailing bag and return the bioindicator (2) or the kit (1) and the accompanying sheet.

14. Use of the bioindicator (2) according to one or more of claims 1 to 7 and/or a kit (1) according to one or more of claims 8 to 12 for the microbiological testing of washing machines.

## Revendications

1. Bioindicateur (2) pour les tests microbiologiques des lave-linges, dans lequel
- le bioindicateur (2) comprend un support planaire (9),
- le support (9) contient des bactéries et/ou des champignons comme germes de référence, dans lequel
- le support est enfermé par une membrane semi-perméable (10)
**caractérisé en ce que**
la membrane (10) est une membrane polymère, possède des pores d'une taille comprise entre 0,1 µm et 1 µm, est perméable à un liquide désinfectant et imperméable aux germes de référence et est fermée à l'extérieur par une soudure autour du support, dans lequel la membrane polymère a une épaisseur comprise entre 0,005 mm et 1 mm, est souple et possède une stabilité mécanique qui permet l'utilisation du bioindicateur sans couvercle de protection dans un cycle de lavage pour les tests microbiologiques des lave-linges, et dans lequel le support a une épaisseur inférieure à 1 mm.

2. Bioindicateur (2) selon la revendication 1,
**caractérisé en ce que**
la membrane (10) est hydrophobe.

3. Bioindicateur (2) selon l'une ou plusieurs des revendications précédentes,
**caractérisé en ce que**
le bioindicateur (2) est planaire.

4. Bioindicateur (2) selon l'une ou plusieurs des revendications précédentes,
**caractérisé en ce que**
le support (9) est perméable à un liquide désinfectant des deux côtés.

5. Bioindicateur (2) selon l'une ou plusieurs des revendications précédentes,
**caractérisé en ce que**
les germes de référence sont sélectionnés parmi le groupe Enterococcus faecium et/ou Staphylococcus aureus et/ou les germes de référence sur le support (9) ont une densité de germes allant jusqu'à 10⁷.

6. Bioindicateur (2) selon l'une ou plusieurs des revendications précédentes,
**caractérisé en ce que**
la membrane (10) a des pores d'une taille < 1 µm, de préférence < 0,6 µm.

7. Bioindicateur (2) selon l'une ou plusieurs des revendications précédentes,
**caractérisé en ce que**
la membrane polymère semi-perméable est choisie parmi le groupe comprenant la polysulfone, la polyéthersulfone, les silicones, le polyamide, le polyamide-imide, le polyamide urée, le polycarbonate, les zéolites, le polyacrylonitrile, le polyéthylène, le polypropylène, le polytétrafluoroéthylène, le fluorure de polyvinylidène, le chlorure de polyvinyle et/ou le polypipérazinamide.

8. Kit (1) comprenant de 2 à 16 bioindicateurs (2) selon une ou plusieurs des revendications précédentes, ainsi qu'une fiche d'application, une fiche d'accompagnement et/ou au moins une pochette d'expédition, dans lequel les bioindicateurs individuels (2) du kit sont combinés dans un élément planaire.

9. Kit selon la revendication 8
**caractérisé en ce que**
le kit contient 2, 4 ou 10 bioindicateurs (2).

10. Kit (1) selon la revendication 8 ou 9, comprenant deux bioindicateurs (2),
**caractérisé en ce que**
un premier bioindicateur (2) contient des germes de référence d'une espèce, de préférence Enterococcus faecium et un second bioindicateur (2) contient des germes de référence d'une seconde espèce, de préférence Candida albicans et/ou Staphylococcus aureus.

11. Kit (1) selon la revendication 8, comprenant 10 bioindicateurs (2),
**caractérisé en ce que**
5 bioindicateurs (2) contient des germes de référence d'une espèce, de préférence Enterococcus faecium, à différents niveaux logarithmiques, et 5 bioindicateurs (2) contient des germes de référence d'une seconde espèce, de préférence Candida albicans et/ou Staphylococcus aureus, à différents niveaux logarithmiques.

12. Kit (1) selon la revendication 8,
**caractérisé en ce que**
2 à 8, de préférence 3 à 6, et de manière particulièrement préférée 4 à 6, et en particulier 5 bioindicateurs (2) contiennent des germes de référence du groupe Enterococcus faecium et 2 à 8, de préférence 3 à 6, de manière particulièrement préférée 4 à 6, et en particulier 5 bioindicateurs (2) contiennent des germes de référence du groupe Staphylococcus aureus, et dans lequel le nombre de bioindicateurs (2) ayant des germes de référence d'un groupe correspond au nombre de bioindicateurs (2) de l'autre groupe.

13. Procédure pour les tests microbiologiques des lave-linges, comprenant les étapes suivantes
- Retrait d'au moins un bioindicateur (2) selon une ou plusieurs des revendications 1 à 8 ou d'un kit (1) selon une ou plusieurs des revendications précédentes 9 à 13, d'une pochette d'expédition,
- Chargement du lave-linge à tester avec l'au moins un bioindicateur (2) ou le kit (1) ainsi qu'un article à désinfecter et/ou à laver,
- Remplissage de la fiche d'accompagnement,
- Retrait et de préférence séchage d'au moins un bioindicateur (2) ou du kit (1),
- Placement de l'au moins un bioindicateur (2) ou du kit (1) et de la fiche d'accompagnement dans une pochette d'expédition et renvoi du bioindicateur (2) ou du kit (1) et de la fiche d'accompagnement.

14. Utilisation du bioindicateur (2) selon une ou plusieurs des revendications 1 à 7 et/ou d'un kit (1) selon une ou plusieurs des revendications 8 à 12 pour les tests microbiologiques des lave-linges.
